(19) Europäisches Patentamt
European Patent Office
Office européen des brevets

(11) EP 1 500 385 B1

(12) **FASCICULE DE BREVET EUROPEEN**

(45) Date de publication et mention de la délivrance du brevet:
**25.06.2008 Bulletin 2008/26**

(21) Numéro de dépôt: **03712904.6**

(22) Date de dépôt: **25.03.2003**

(51) Int Cl.:
***A61K 8/04*** *(2006.01)* ***A61K 8/73*** *(2006.01)*
***A61Q 19/00*** *(2006.01)* ***A61Q 17/04*** *(2006.01)*
***A61Q 1/00*** *(2006.01)* ***A61Q 5/00*** *(2006.01)*

(86) Numéro de dépôt international:
**PCT/JP2003/003598**

(87) Numéro de publication internationale:
**WO 2003/082225 (09.10.2003 Gazette 2003/41)**

(54) **PROCEDE DE STABILISATION DE MOUSSE POUR MOUSSE COSMETIQUE**

VERFAHREN ZUR SCHAUMSTABILISIERUNG FÜR EINEN KOSMETIKSCHAUM

METHOD OF FOAM STABILIZATION FOR FOAM COSMETIC

(84) Etats contractants désignés:
**FR**

(30) Priorité: **28.03.2002 JP 2002092441**

(43) Date de publication de la demande:
**26.01.2005 Bulletin 2005/04**

(73) Titulaire: **Hakuto Co., Ltd**
**Shinjuku-ku,**
**Tokyo 160-8910 (JP)**

(72) Inventeurs:
- **NOHATA, Yasuhiro,**
**c/o Yokkaichi Laboratory**
**Yokkaichi-shi,**
**Mie 510-0007 (JP)**
- **KUROMIYA, Tomomi,**
**c/o Yokkaichi Laboratory**
**Yokkaichi-shi,**
**Mie 510-0007 (JP)**

(74) Mandataire: **Pautex Schneider, Nicole Véronique et al**
**Novagraaf International SA**
**25, Avenue du Pailly**
**1220 Les Avanchets - Geneva (CH)**

(56) Documents cités:
**EP-A1- 0 037 999**
**WO-A2-94/12145**
**JP-A- 5 085 925**
**JP-A- 2000 355 517**
**JP-A- 2001 226 250**
**JP-A- 2001 247 442**
**JP-A- 2001 247 443**
**JP-A- 2002 121 538**
**JP-A- 2003 055 641**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 500 385 B1

## Description

### Domaine technique de l'invention

La présente invention concerne des produits cosmétiques contenant une mousse finement dispersée, en particulier des produits cosmétiques en mousse qui présentent une excellente stabilité de mousse et un toucher léger lorsqu'ils sont pris à la main ou appliqués sur la peau, par rapport à des produits cosmétiques classiques.

### Art antérieur

En général, on a de préférence utilisé des produits cosmétiques de façon à présenter un toucher léger dépourvu de caractère collant et une bonne capacité d'étalement sans traînée lorsqu'ils sont pris à la main ou appliqués sur la peau. Par conséquent, des produits cosmétiques contenant une mousse fine ont été proposés. Des exemples de ces produits cosmétiques englobent les produits cosmétiques de soin de la peau tels que les crèmes pour la peau y compris les crèmes de nettoyage, les crèmes pour le visage, les crèmes pour les mains et les crèmes pour le corps, et les plâtres (les plates en gel) ; les produits de maquillage tels que les crèmes de fond de teint, les rouges à lèvre, les produits de maquillage pour les yeux et les fards à joues ; et les produits de traitement capillaire tels que les crèmes capillaires.

Dans ces produits cosmétiques, habituellement, une mousse fine est formée et stabilisée à l'aide d'un tensioactif, et le mouvement et la coalescence de la mousse fine sont empêchés par un agent épaississant en combinaison avec le tensioactif, de sorte que la mousse fine est conservée et stabilisée.

Habituellement, après avoir été fabriqués et stockés, les produits cosmétiques en mousse sont transportés vers des entrepôts de livraison, sont livrés aux magasins, sont présentés dans des casiers d'exposition et sont ensuite vendus. La mousse fine dans les produits cosmétiques doit être maintenue de façon stable pendant une durée prolongée jusqu'à ce que les consommateurs utilisent les produits cosmétiques après achat. En outre, la sensation des produits cosmétiques lorsqu'ils sont touchés à la main ou appliqués sur la peau doit être également conservée sans être modifiée.

Toutefois, étant donné que la mousse fine est dispersée dans les produits cosmétiques en mousse pour entraîner une différence de masse volumique entre la mousse fine et les substances avoisinantes, la mousse fine est susceptible de flotter progressivement et de coalescer les unes avec les autres. Par conséquent, il apparaît une différence de la taille de la mousse qui détériore l'aspect externe des produits cosmétiques produits, et une différence de sensation de ceux-ci lors de l'utilisation, de sorte que les avantages des produits cosmétiques produits, ainsi que la confiance des consommateurs en ceux-ci, sont grandement réduits. Par conséquent, les produits cosmétiques en mouse n'ont été appliqués qu'à des produits cosmétiques spécifiques, tels que les produits cosmétiques de type aérosol adaptés pour faire appel à de l'air lors de l'emploi.

Par conséquent, divers procédés améliorés pour les produits cosmétiques en mousse ont été proposés. La publication de la demande de brevet japonais (JP-B) N˚ 63-23962 décrit des produits cosmétiques battus obtenus par mélange d'un agent huileux spécifique dans ceux-ci pour fabriquer une chasse d'air, la publication nationale de la demande de brevet japonais N˚ 8-503936 décrit une composition cosmétique dans laquelle une mousse fine ayant une taille réduite inférieure à 20 $\mu$m est utilisée pour la mélanger de façon stable, et la demande de brevet japonais ouverte à l'inspection publique (JP-A) N˚ 2000-355517 décrit des produits cosmétiques en mousse dans lesquels une gomme de gellane est utilisée pour stabiliser la mousse.

Toutefois, même lorsque la quantité de tensioactif est accrue, la mousse fine ne peut pas être suffisamment stabilisée et les produits cosmétiques deviennent collants lors de l'utilisation, de sorte qu'ils sont moins préférables. D'autre part, lorsqu'un agent épaississant et dispersant à base d'un polysaccharide est utilisé, la mousse fine ne peut pas être suffisamment stabilisée et les produits cosmétiques deviennent collants et présentent une sensation lourde avec une plus faible capacité d'étalement lors de l'emploi, de sorte qu'ils sont moins préférables.

Par conséquent, on n'a pas obtenu de produits cosmétiques en mousse satisfaisants qui sont capables de disperser de façon stable une certain nombre de mousse fine pendant une durée prolongée de façon à conserver une qualité stable des produits cosmétiques en mousse, et présentant une sensation légère et une bonne capacité d'étalement lors de l'utilisation.

### Description de l'invention

La présente invention a été réalisée dans ces circonstances et a pour but de fournir un procédé de stabilisation d'une mouse fine dans des produits cosmétiques en mousse pendant une durée prolongée, tout en présentant une sensation légère et une bonne capacité d'étalement lorsqu'ils sont pris à la main ou lorsqu'ils sont appliqués sur la peau.

Les présents inventeurs ont mené des études poussés pour découvrir qu'en utilisant un polysaccharide spécifique, une mousse fine peut être dispersée de façon stable dans les produits cosmétiques et qu'une sensation légère peut être obtenue lors de l'utilisation et, sur la base de leurs découvertes, ils ont réalisé la présente invention.

L'invention selon la revendication 1 fournit un procédé de stabilisation d'une mousse dans des produits cosmétiques en mousse, qui comprend une étape de mélange d'un polysaccharide constitué au moins de glucose, de fucose, d'acide glucuronique et de rhamnose en tant que monosaccharides dans les produits cosmétiques en mousse.

En mélangeant le polysaccharide décrit ci-dessus, on peut obtenir une composition cosmétique en moussé dans laquelle la mousse est dispersé de façon stable pendant une durée prolongée avec une teneur en polysaccharide plus faible, par rapport au cas où un polysaccharide classique est utilisé, et une sensation rafraîchissante peut être obtenue lors de l'utilisation, ce qui est préférable.

L'invention selon la revendication 2 fournit le procédé de stabilisation d'une mousse dans des produits cosmétiques en mousse selon la revendication 1, dans lequel le polysaccharide est un produit d'un micro-organisme de la souche Alcaligenes latus B-16.

En utilisant le micro-organisme de la souche Alcaligenes latus B-16, on peut obtenir plus efficacement le polysaccharide en tant que produit.

L'invention selon la revendication 3 fournit le procédé de stabilisation d'une mousse dans des produits cosmétiques en mousse selon la revendication 1 ou 2, dans lequel le polysaccharide décrit ci-dessus est mélangé dans une proportion de 0,01 à 0,5 % en poids, convertie en fraction solide séchée, par rapport à la quantité totale des produits cosmétiques.

Avec le procédé de la présente invention, une mousse fine dans les produits cosmétiques en mousse est dispersée de façon stable et est conservée pendant une durée prolongée et, en raison de la mousse dans lés produits cosmétiques, une sensation légère et une bonne capacité d'étalement peuvent être obtenues lors de l'utilisation, et cette sensation légère peut être conservée pendant une durée prolongée, contribuant de ce fait à l'amélioration de la qualité des produits cosmétiques en mousse.

Le meilleur mode de réalisation pour mettre en oeuvre l'invention

Le procédé de stabilisation d'une mousse dans des produits cosmétiques en mousse selon la présente invention est un procédé de mélange d'un polysaccharide spécifique dans les produits cosmétiques en mousse pour y stabiliser la mousse fine, et pour conserver un bon état de dispersion de la mousse fine pendant une durée prolongée tout en présentant une sensation légère et une bonne capacité d'étalement lors de l'utilisation.

Des exemples des produits cosmétiques en mousse englobent des produits cosmétiques aqueux qui contiennent de façon stable une grande quantité de mousse fine (de type solution aqueuse, de type soluble dans les composants huileux, de type gel aqueux, suspension aqueuse), et des produits cosmétiques en émulsion (type crème huile dans eau, de type émulsion huile dans eau, de type gel huile dans eau, de type crème eau dans huile, de type gel eau dans huile) et englobent aussi les produits cosmétiques sous forme de pâte. En outres, les produits cosmétiques aqueux en mousse sont préparés par fabrication de produits cosmétiques aqueux qui contiennent une mousse fine, les produits cosmétiques en mouse en émulsion huile dans eau sont préparés par émulsification de composants aqueux, tels que des composants retenant l'humidité, et des composants huileux avec un tensioactif, afin de contenir la mousse fine. Les produits cosmétiques en mousse de la présente invention sont sous forme d'une solution, sous forme d'une crème, sous forme d'une pâte, et ils contiennent de façon stable une mousse. Des exemples de ceux-ci englobent les produits cosmétiques de base tels qu'une crème de nettoyage du visage, une crème de nettoyage (mousse), une crème de rasage, une mouse de rasage, une lotion, un pansement, une crème de massage, une lotion laiteuse conservant l'humidité, une crème conservant l'humidité et une crème pour les lèvres ; les produits cosmétiques de finition, tels que le fond de teint, l'ombre pour les yeux, le crayon pour les yeux, le mascara et le rouge à lèvre ; les produits cosmétiques capillaires tels que la gelée pour les cheveux, la crème capillaire, la mousse capillaire, le shampoing, un produit de rinçage, un traitement capillaire, un produit de conditionnement capillaire, un liquide capillaire et une lotion fixante ; les produits cosmétiques de type parfum, tels que le parfum (parfum et produit parfumé), l'eau de parfum (eau de cologne), l'eau de toilette (parfum de toilette, et produit parfumé de toilette) et l'eau de cologne (Cologne, Cologne frais) ; les produits cosmétiques contre les coups de soleil tels qu'une gelée solaire, une crème solaire et une lotion laiteuse solaire.

Les composants huileux pour les produits cosmétiques en mousse ne sont pas spécifiquement limités, tant qu'ils sont habituellement utilisés pour les produits cosmétiques. Ils peuvent être convenablement choisis en fonction des compositions des produits cosmétiques et ne sont pas spécifiquement limités indépendamment de leurs origines telles que les huiles naturelles et synthétiques, et leurs formes tels que des solides, des semi-solides et des liquides.

Par exemple, les agents huileux englobent les hydrocarbures, les cires, les acides gras, un alcool supérieur, les esters d'acides gras, les esters d'acides organiques, les glycérides, les hydrocarbures de fluoration, les huiles de silicone, etc. Plus spécifiquement, des exemples des hydrocarbures englobent le squalane, le squalène, la cérésine, la paraffine, la cire de paraffine, la paraffine liquide, le pristane, le polyisobutyène, la cire microcristalline, la vaseline, etc. Des exemples de cires englobent la cire d'abeille, la cire de carnauba, la cire de candélilla, la cire de requin, etc., des exemples d'huiles animales englobent la suif de boeuf, la graisse de jambe de bétail, la graisse d'os de bétail, la suif de boeuf durcie, une huile hydrogénée, l'huile de tortue de mer, le lard, la graisse de cheval, l'huile de vison, l'huile de

foie, l'huile de jaune d'oeuf, etc., des exemples de dérivés de lanoline englobent la lanoline, la lanoline liquide, la lanoline réduite, l'alcool de lanoline, la lanoline dure, l'acétate de lanoline, l'acide gras isopropylique de lanoline, l'alcooléther de lanoline et de POE, l'acétate d'alcool de lanoline et de POE, l'acide gras de lanoline de polyéthylèneglycol, l'alcooléther de lanoline de POE hydrogéné, etc., des exemples d'acides gras englobent l'acide laurique, l'acide myristique, l'acide palmitique, l'acide stéarique, l'acide béhénique, l'acide undécylénique, l'acide oléique, l'acide arachidonique, l'acide docosahexanoique (DHA), l'acide isostéarique, l'acide 1,2-hydroxystéarique, etc., des exemples d'alcools supérieurs englobent l'alcool laurylique, l'alcool myristylique, l'alcool palmitylique, l'alcool stéarylique, l'alcool béhénylique, l'alcool hexadécylique, l'alcool oléylique, l'alcool isostéarylique, l'hexyldodécanol, l'octyldodécanol, l'alcool cétostéarylique, le 2-décyltétradécinol, le cholestérol, le phytostérol, le sitostérol, le lanostérol, le cholestéroléther de POE, le monostéarylglycérineéther (l'alcool batylique), etc., des exemples d'esters d'acides gras englobent l'adipate de diisobutyle, l'adipate de 2-hexyldécyle, l'adipate de 2-heptylundécyle, le monoisostéarate de N-alkylglycol, l'isostéarate d'isocétyle, le triisostéarate de triméthylolpropane, le di-2-éthylhexanoate d'éthylèneglycol, le 2-éthylhexanoate de cétyle, le tri-2-éthylhexanoate de triméthylolpropane, le tétra-2-éthylhexanoate de pentaérythritol, l'éthylhexanoate de cétyle, l'octyldodécylester de gomme, l'oléate d'oléyle, l'oléate de dodécyloctyle, l'oléate de décyle, le dicaprate de néopentylglycol, le citrate de triéthyle, le succinate de 2-éthylhexyle, l'acétate d'amyle, l'acétate d'éthyle, l'acétate de butyle, le stéarate d'isocétyle, le stéarate de butyle, le sébacate de diisopropyle, le sébacate de di-2-éthylhexyle, le lactate de cétyle, le lactate de myristyle, le palmitate d'isopropyle, le palmitate de 2-éthylhexyle, le palmitate de 2-hexyldécyle, le palmitate de 2-heptylundécyle, le 12-hydroxystéarate de cholestéryle, l'ester d'acide gras de dipentaérythritol, le myristate d'isopropyle, le myristate d'octyldodécyle, le myristate de 2-hexyldécyle, le myristate de myristyle, le diméthyloctanate d'hexyldécyle, le laurate d'éthyle, le laurate d'hexyle, etc., des exemples d'ester d'acide aminé englobent le 2-octyldodécylester d'acide N-lauroyl-L-glutamique, des exemples d'ester d'acide organique englobent le malate de di-isostéaryle, etc., des exemples de glycérides englobent l'acétylglycéride, le tri-isooctanoate de glycéride, le tri-isostéarate de glycéride, le tri-isopalmitate de glycéride, le tri-2-éthylhexanoate de glycéride, le monostéarate de glycéride, le di-2-heptylundécanoate de glycéride, le trimyristate de glycéride, etc., et des exemples d'hydrocarbure fluoré englobent le polyperfluoroéther, la perfluorodécaline, le perfluorooctane, etc.

Des exemples de l'huile de silicone englobent le diméthylpolysiloxane, l'éthylméthylpolysiloxane, le diéthylpolysiloxane, le méthylhydrogénopolysiloxane, le méthylphénylpolysiloxane, un organopolysiloxane modifié par un polyéther tel qu'un copolymère diméthylsiloxaneméthyl(polyoxyéthylène)siloxane et un copolymère diméthylsiloxane-méthyl (polyoxyéthylènepolyoxypropylène)siloxane, un diméthylpolysiloxane cyclique tel qu'un copolymère diméthylsiloxanealcoxy(comportant 4 à 12 atomes de carbone)-méthylsiloxane, l'octaméthylcyclotétrasiloxane, le décaméthylcyclopentasiloxane, le dodécaméthylcyclohexasiloxane, un organosiloxane modifié par du fluor tel qu'un copolymère fluorométhylsiloxanediméthylsiloxane, un organopolysiloxane modifié par un fluoroalkylpolysiloxane tel qu'un copolymère fluorométhylsiloxane-polyoxyéthylèneméthylsiloxane et un copolymère fluorométhylsiloxane-polyoxyéthylènepolyoxypropylèneméthylsiloxane, un organopolysiloxane à extrémité ou à chaîne latérale modifiée tel qu'une substance modifiée par un diméthylpolysiloxane dans laquelle un groupe hydroxyle est introduit à une extrémité de celle-ci, et un copolymère hydroxyméthylsiloxane-diméthylpolysiloxane dans lequel un groupe hydroxyle est partiellement introduit dans une chaîne latérale, et un aminoorganopolysiloxane modifié tel qu'un polymère diméthylaminobutylméthylsiloxane-diméthylsiloxane ayant un groupe dialkylaminoalkyle dans une chaîne latérale de celui-ci, etc. Habituellement, l'huile de silicone ayant une viscosité de 100 000 (mPa.s ; 25˚C) ou moins est choisie comme composant de la composition cosmétique. La teneur de cette huile de silicone s'échelonne de 0,1 à 80 % en poids (par rapport à la quantité totale de la composition cosmétique), et la teneur préférable de celle-ci s'échelonne de 0,5 à 50 % en poids (dans la suite, "% en poids" sera appelé %).

Ces composants huileux peuvent être utilisés seuls ou en combinaison de deux d'entre eux ou plus.

La quantité des composants huileux peut être correctement déterminée en tenant compte de l'effet de conservation de l'humidité recherché, et elle s'échelonne habituellement de 0,1 à 60 %, mieux encore de 1 à 30 %.

Le polysaccharide à utiliser dans la présente invention est le polysaccharide constitué d'un moins du fucose, du glucose, de l'acide glucuronique et de la rhamnose en tant que monosaccharides constitutifs et, comme représenté par la formule (1) suivante, il a de préférence des chaînes principales avec un construction de répétition constituée de fucose, d'acide glucuronique et de rhamnose, où un fucose diverge d'un glucose dans la chaîne principale.

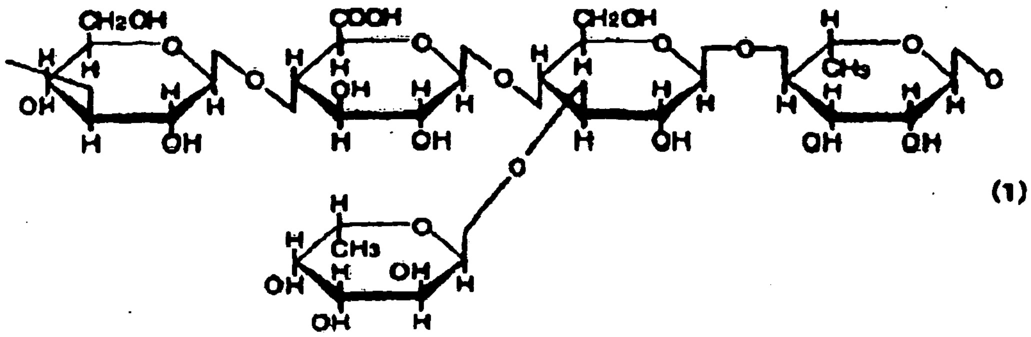

(1)

Le polysaccharide représenté par la formule (1) peut être obtenu, par exemple, sous forme d'un produit d'un micro-organisme de la souche Alcaligenes latus B-16 (FERM BP-2015). Le micro-organisme de la souche Alcaligenes latus B-16 est cultivé par un procédé de culture de microbes habituel et, après la culture, des solvants organiques tels que l'acétone, l'éthanol et l'alcool isopropylique, sont ajoutés au liquide de culture. Le polysaccharide précipite ainsi sous forme d'une substance insoluble. La précipitation du polysaccharide est séparé, afin de donner un polysaccharide.

Généralement, les micro-organismes produisent deux types ou plus de polysaccharides. D'autres types de polysaccharide que le polysaccharide de la présente invention peuvent être incorporés, à condition qu'ils n'altèrent pas les effets de la présente invention. Par exemple, il a été prouvé que les polysaccharides produits par un micro-organisme de la souche Alcaligenes latus B-16 (appelé "polysaccharide B-16" dans la suite) comprennent au moins deux types de polysaccharides, et le rapport molaire des monosaccharides constitutifs des polysaccharides qui sont séparés du liquide de culture est : fucose : glucose : acide glucuronique : rhamnose = 1 : (0,5 à 4) : (0,5 à 2) : (0,5 à 2).

Lorsque ces deux types de polysaccharides sont séparés l'un de l'autre, un type de polysaccharide est un polysaccharide ayant une construction dans laquelle un fucose diverge d'un glucose dans les chaînes principales avec une construction répétée, qui est constituée de glucose, d'acide glucuronique et de rhamnose, comme le montre la formule (1), et un autre type de polysaccharide est un polysaccharide duquel un motif de répétition est constitué de fucose et de mannose. Le premier est le polysaccharide de la présente invention, a un rapport de constitution du fucose, glucose, acide glucuronique et rhamnose qui est de 1 : 2 : 1 : 1, et qui est un composant de masse moléculaire élevée ayant une masse moléculaire d'environ $10^9$ [voir le Japan Agricultural Chemistry Society, 1998 the Year Large Meeting, résumé, page 371]. Le deuxième est un polysaccharide ayant une construction de répétition de fucose et de mannose de 1 : 1, et est un composant de faible masse moléculaire ayant une masse moléculaire de $10^3$ à $10^7$ [voir Y. Nohata, J. Azuma, R. Kurane, Carbohydrate Research 293, (1996) 213 à 222]. Ce composant de faible masse moléculaire ne se situe pas dans le cadre du polysaccharide de la présente invention, mais il n'altère par l'effet de stabilisation de la présente invention, de sorte qu'il peut être contenu dans la composition cosmétique de la présente invention. Ce polysaccharide est maintenant vendu sur le marche sous la marque de commerce Alcasealan [marque de commerce, INCIname : Alcalignes Polysaccharides, fabriqué par HAKUTO CO., Ltd.].

Le polysaccharide B-16 présente une excellente stabilité de dispersion d'une mousse, par rapport à des polymères naturels solubles dans l'eau classiques tels que la gomme xanthane et les polymères synthétiques solubles dans l'eau tels que le poly(alcool vinylique), et l'effet de celui-ci est moins affecté par les sels existant conjointement, par la température et le pH sans diminution de l'effet au cours du temps. Par conséquent, la stabilité à long terme de la mousse fine est notablement améliorée avec une grande augmentation de la qualité des produits cosmétiques en mousse en aérosol existants. Par ailleurs, dans les produits cosmétiques en mousse tels que les crèmes retenant l'humidité, les crèmes solaires, les crèmes de fond de teint et le rouge à lèvre, etc., la qualité telle que la stabilité de la mousse fine, qui n'a pas été entièrement atteinte de façon classique, est grandement améliorée. De plus, le polysaccharide B-16 a une capacité d'absorption de l'eau appropriée et une faible capacité de rejet de l'eau, par rapport à des polymères naturels et synthétiques solubles dans l'eau classiques. La dispersion aqueuse du polysaccharide B-16 a également une faible viscosité avec une sensation rafraîchissante. Par conséquent, les produits cosmétiques mélangés avec le polysaccharide B-16 donnent une sensation légère lorsqu'ils sont mis sur les doigts ou étalés sur la peau. Par conséquent, une sensation légère agréable qui n'a pas été atteinte par les produits cosmétiques en mousse classiques utilisant des polymères naturels et synthétiques solubles dans l'eau classiques, peut être obtenue à l'emploi.

La quantité mélangé du polysaccharide pour stabiliser la mousse dans les produits cosmétiques en mousse de l'invention s'échelonne habituellement de 0,01 à 0,5 % en poids ("% en poids" sera appelé "%" dans la suite), et elle s'échelonne de préférence de 0,03 à 0,3 %, et mieux encore de 0,05 à 0,1 %, par rapport à la quantité totale des produits cosmétiques, convertie en la fraction solide sèche. Lorsque la quantité mélangée du polysaccharide est inférieure à 0,01 %, l'effet du polysaccharide de l'invention peut ne pas s'exprimer complètement. Lorsque la quantité mélangée du

polysaccharide est supérieure à 0,5 %, l'effet est peu accru et, par conséquent, les avantages économiques sont faibles.

La quantité de la mousse contenue dans les produits cosmétiques de la présente invention s'échelonne de 0,001 à 99 % en volume, de préférence de 5 à 80 % en volume, et mieux encore de 20 à 70 % en volume, par rapport au volume total des produits cosmétiques. Le diamètre moyen de la mousse dans les produits cosmétiques peut être choisi et déterminé en fonction des produits cosmétiques en mousse recherchés, mais elle s'échelonne habituellement de 1 nm à 5 nm, de préférence de 1 à 500 μm, et mieux encore de 10 à 200μ m. Cette gamme peut être choisie en tenant compte des effets résultants tels que la stabilité à long terme, la sensation légère sur les doigts et la bonne capacité d'étalement et la sensation légère sur la peau, et elle est convenablement déterminée en fonction des utilisations des produits cosmétiques.

Des exemples du procédé de fabrication de la mousse mise en jeu dans les produits cosmétiques englobent un procédé de mélange, d'agitation ou de dispersion de gaz comprimés tels que l'air, les composés à base de chlorofluorocarbone, l'azote, le dioxyde de carbone, l'oxygène et l'hélium, et des gaz liquéfiés tels que les gaz de pétrole liquéfiés (GPL) notamment l'éther diméthylique, le propane, le propylène, le n-butane, l'isobutane, le n-butylène, l'isobutylène, le butadiène, le n-pentane, l'isopentane, le n-hexane et l'iso-hexane, tout en faisant barboter ces gaz dans le procédé de production des produits cosmétiques ; un procédé d'agitation utilisant un mélangeur d'agitation ayant une structure qui peut facilement capturer la mousse ; un procédé de mélange et d'agitation avec un agent moussant tel qu'un tensioactif pour générer une mousse dans les produits cosmétiques, etc. Le procédé décrit ci-dessus n'est pas spécifiquement limité mais, habituellement, le procédé d'utilisation du tensioactif en tant qu'agent moussant et de l'agent dispersant et stabilisant une mousse est adopté. Par ailleurs, le procédé de mise des produits cosmétiques dans un récipient avec un agent de pulvérisation (gaz de pulvérisation) en tant que produit d'aérosol, et de génération d'une mousse dans les produits cosmétiques lors de la pulvérisation est adopté.

Tout tensioactif qui est habituellement utilisé pour l'émulsification et la dispersion des produits cosmétiques peut être utilisé, et des exemples de celui-ci englobent les tensioactifs non ioniques, les tensioactifs anioniques, les tensioactifs cationiques et les tensioactifs amphotères.

Des exemples de tensioactifs non ioniques englobent les éthers alkyliques polyalcoxylés, les éthers alkylphényliques polyalcoxylés, les esters d'acides gras polyalcoxylés, les esters d'acides gras de sorbitan polyalcoxylés, les esters d'acides gras de sorbitan, les esters d'acide gras de glycérine polyalcoxylés, les esters d'acide gras de glycérine, les esters d'acide gras de polyglycérine, les huiles de ricin hydrogénées polyalcoxylés, les esters d'acides gras de saccharose, les alkylamines polyalcoxylées, les copolymères séquencés oxyde d'éthylène-oxyde de propylène, etc.

Le polyoxyalkykène dans les tensioactifs non iniques est constitué d'au moins un polyoxyéthylène (appelé "POE" dans la suite), un polyoxypropylène (appelé "POP" dans la suite), et de polyoxybutylène (appelé "POB" dans la suite). Le nombre de moles de polymérisation de chacun de POE, POP et POB est déterminé arbitrairement, par rapport aux caractéristiques d'émulsion souhaitées du tensioactif, et il est habituellement de 3 à 200. De plus, le rapport molaire de polymérisation du POE, POP et POB est déterminé arbitrairement, par rapport aux caractéristiques d'émulsion souhaitées du tensioactif. De préférence, le polyoxyalkylène est constitué de POE et de POP, et un rapport molaire de POE est de 25 % en moles ou plus.

Les éthers alkyliques polyalcoxylés comportant 2 à 4 atomes de carbone sont préparés par addition d'un poly (oxyde d'alkylène) à un alcool saturé ou insaturé, du type à chaîne droite ou de type ramifié, comportant 8 à 30 atomes de carbone. Plus spécifiquement, des exemples des éthers alkyliques polyalcoxylés comportant 2 à 4 atomes de carbone englobent l'octyléther de POE (3 moles), le dodécyléther de POE (5 moles), l'oléyléther de POE (10 moles), le stéaryléther de POE (15 moles), le béhényléther de POE (20 moles), le décyléther de POE (10 moles) et de POP (10 moles), l'isostéaryléther de POE (15 moles) et de POP (2 moles), le cholestanoléther de POE (10 moles), les lanolines hydrogénées de POE (10 mole) et de POP (0 mole), etc.

Les éthers alkylphényliques polyalcoxylés sont préparés par addition de poly(oxyde d'alkylène) à un alkylphénol et à un alcénylphénol de type chaîne droite ou de type ramifié, comportant chacun 1 à 22 atomes de carbone. Plus spécifiquement, des exemples d'éther alkylphényliques polyalcoxylés englobent le méthylphényléther polyéthylé (3 moles), l'octylphényléther de POE (5 moles), le nonylphényléther de POE (10 moles), le dodécylphényléther de POE (15 moles), etc.

Les esters d'acides gras polyalcoxylés sont préparés par addition d'un poly(oxyde d'alkylène) à un acides gras saturé de type chaîne droite ou de type ramifié ou à un acide gras insaturé comportant 8 à 22 atomes de carbone. Plus spécifiquement, des exemples des esters d'acides gras polyalcoxylés englobent l'ester d'acide octanique de POE (3 moles), l'ester d'acide décanique de POE (5 moles), l'ester d'acide dodécanique de POE (10 moles), l'ester d'acide stéarique de POE (15 moles), l'ester d'acide béhénique de POE (20 moles), l'ester d'acide isostéarique de POE (15 moles), l'ester d'acide oléique de POE (15 moles) et de POP (5 moles), etc.

Les esters d'acides gras de sorbitan polyalcoxylés sont préparés par addition de poly(oxyde d'alkylène) à du sorbitol et à un acide gras saturé de type chaîne droite ou de type ramifié ou à un acide gras insaturé comportant 8 à 22 atomes de carbone. Plus spécifiquement, des exemples des esters d'acides gras de sorbitan polyalcoxylés englobent le monolaurate de sorbitan de PEO (5 moles), le trilaurate de sorbitan de PEO (20 moles), le monostéarate de sorbitan

de POE (20 moles), le sesquistéarate de sorbitan de POE (20 moles), le tristéarate de sorbitan de POE (20 moles), le monooléate de sorbitan de POE (20 moles), etc.

Les esters d'acides gras de sorbitan sont des esters constitués de sorbitol et d'un acide gras saturé de type chaîne droite ou de type ramifié ou à un acide gras insaturé comportant 8 à 22 atomes de carbone. Plus spécifiquement, des exemples des esters d'acides gras de sorbitan englobent le monolaurate de sorbitan, le monopalmitate de sorbitan, le monostéarate de sorbitan, le monoisostéarate de sorbitan, le monooléate de sorbitan, le sesquioléate de sorbitan, le trioléate de sorbitan, le diglycérolsorbitan d'acide penta-2-éthylhexylique, le diglycérolsorbitan d'acide tétra-2-éthylhexylique, etc.

Les esters d'acides gras de glycérine polyalcoxylés sont des esters d'addition constitués de glycérine, d'un acide gras saturé de type chaîne droite ou de type ramifié ou d'un acide gras insaturé comportant 8 à 22 atomes de carbone et de poly(oxyde d'alkylène). Plus spécifiquement, des exemples des esters d'acides gras de glycérine polyalcoxylés englobent le monolaurate de glycérol de POE (5 moles), le monostéarate de glycérol de POE (10 moles), le distéarate de glycérol de POE (15 moles), le dioléate de glycérol de POE (20 moles) et de POP (5 moles), etc.

Les esters d'acides gras de glycérine sont des esters constitués de glycérine et d'un acide gras saturé de type chaîne droite ou de type ramifié ou à un acide gras insaturé comportant 8 à 22 atomes de carbone. Plus spécifiquement, des exemples des esters d'acides gras de glycérine englobent le monolaurate de glycéryle, le sesquilaurate de glycéryle, le trilaurate de glycéryle, le monostéarate de glycéryle, le sesquistéarate de glycéryle, le tristéarate de glycéryle, le monooléate de glycéryle, le sesquioléate de glycéryle, le trioléate de glycéryle, le monoglycéride d'acide gras d'huile de graine de coton, le monoétucate de glycéryle, le pyroglutamate d'$\alpha,\alpha'$-glycéryle, l'ester d'un mélange d'acides gras saturés comportant 8 à 12 atomes de carbone et de glycérine et l'ester de l'acide stéarique, de l'acide malique et de glycérine, etc.

Des exemples d'esters d'acide gras de polyglycérine englobent le polyglycérolester d'acide hydroxystéarique condensé, le polyglycérolester d'acide ricinoléique condensé, etc., et des exemples d'huiles de ricin hydroxylées polyalcoxylées englobent l'huile de ricin de POE (10 moles), l'huile de ricin hydrogénée de POE (15 moles), le monoisostéarate d'huile de ricin hydrogénée de POE (15 moles), le triisostéarate d'huile de ricin hydrogénée de POE (20 moles), le diester monopyroglutamate-monoisostéarate d'huile de ricin hydrogénée de POE (20 moles), le malénate d'huile de ricin hydrogénée de POE (20 moles), etc.

Les esters d'acides gras de saccharose sont des esters constitués de saccharose et d'un acide gras saturé de type chaîne droite ou de type ramifié ou à un acide gras insaturé comportant 8 à 22 atomes de carbone. Plus spécifiquement, des exemples des esters d'acides gras de saccharose englobent le béhénate de saccharose, le stéarate de saccharose, le palmitate de saccharose, le miristate de saccharose, le laurate de saccharose, l'érucate de saccharose, l'oléate de saccharose, etc.

Les alkylamines polyalcoxylées sont préparées par addition de poly(oxyde d'alkylène) à une amine primaire ou secondaire comportant 3 à 22 atomes de carbone et, plus spécifiquement, des exemples de celles-ci englobent la didodécylamine de POE (5 moles), la dodécylamine de diPOE (10) et de POP (3), la distéarylamine de POE (10 moles), la stéarylamine de diPOE (10 moles), l'oléylamine de diPOE (15 moles), la béhénylamine de diPOE (17 moles), etc.

Les copolymères oxyde d'éthylène-oxyde de propylène sont des copolymères obtenus par polymérisation d'oxyde d'éthylène et d'oxyde de propylène dans un rapport molaire de 1:9 à 9:1 de façon à obtenir une masse moléculaire d'environ 500 à 50 000.

De plus, des exemples de tensioactifs anioniques englobent les sels d'acides gras, les sels d'alkylsulfate, les sels d'alcénylsulfate, les sels d'alkylphénylsulfate et les sels d'alcénylphénylsulfate, les sels d'alkylphényl-éthersulfate polyalcoxylés et les sels d'alcénylphényl-éthersulfate polyalcoxylés, les sels de sulfosuccinate de (di)alkyle, les sels de N-acylaminé (les acyl-N-méthyltaurines), les sels d'alkylbenzènesulfonate, les sels d'alkylnaphtalènesulfonate, la polycondensation de formaline de sels de naphtalènesulfonate, etc. Des exemples préférables de sels métalliques englobent les sels de sodium, les sels de potassium et les sels d'ammonium.

Les sels d'acides gras sont des sels métalliques d'un acide gras saturé de type à chaîne droite ou de type ramifié ou d'un acide gras insaturé comportant 8 à 30 atomes de carbone et, plus spécifiquement, des exemples de ceux-ci englobent l'octylate de sodium, le décanoate de sodium, le dodécanoate de sodium, le tétradécanate de sodium, le stéarate de sodium, l'isostéarate de sodium, l'oléate de sodium, le linolénate de sodium, l'édétate de sodium, etc.

Les alkylsulfates et les alcénylsulfates sont des alkylsulfates et des alcénylsulfates saturés de type à chaîne droite ou de type ramifié ou insaturés comportant 8 à 30 atomes de carbone et, plus spécifiquement, des exemples de ceux-ci englobent l'octylsulfate de sodium, le décylsulfate de sodium, le dodécylsulfate de sodium, l'alkylsulfate de palm de sodium, le stéarylsulfate de sodium, l'isostéarylsulfate de potassium, l'oléylsulfate d'ammonium, le béhénylsulfate d'ammonium, etc.

Les alkylphényléthersulfates et les alcénylphényléthersulfates sont des sels d'ester sulfurique d'un produit d'addition d'un groupe phényle contenant un groupe alkyle ou un groupe alcényle, qui comporte 1 à 22 atomes de carbone et des chaînes droites ou des chaînes ramifiées, et d'un polyoxylkylèneglycol comportant 2 à 4 atomes de carbone et, plus spécifiquement, des exemples de ceux-ci englobent le tosylsulfate de POE (3 moles) de sodium,

l'octylphénylsulfate de POE (5 moles) de sodium, le nonylphénylsulfate de POE (10 moles) de potassium, le décylphénylsulfate de POE (10 moles), l'octadécénylsulfate de POE (15 moles) de potassium, l'isooctadécylsulfate de POE (15 moles) et de POP (5 moles) de potassium, etc.

Des exemples de sels de (di)alkylsulfosuccinates englobent le dioctylsulfosuccinate de sodium, le di-2-éthylhexylsulfosuccinate de sodium, le monolaurylmonoéthanolamidosulfosuccinate de sodium polyéthoxylé, le laurylglycolsulfosuccinate de sodium polypropylé, etc.

Les sels d'acides N-acylaminés sont des acyl-N-méthyltaurines et, plus spécifiquement, des exemples de ceux-ci englobent les sels d'amidosulfonate d'acide aminé gras supérieur tels que la lauroylsarcosine de sodium, la N-myristoyl-N-méthyltaurine de sodium, la méthyltaurine d'acide gras de coprah de sodium, la laurylméthyltaurine de sodium, etc., et un glutamate de N-acyle tel que le glutamate de N-lauryle monosodique, le glutamate N-stéaroyle disodique, le glutamate de N-myristyle L-monosodique, etc. Des exemples de sels alkylbenzènesulfonate englobent le dodécylbenzènesulfonate de sodium, le dodécybenzènesulfonate de potassium, le dodécylbenzènesulfonate d'ammonium, etc. Ces substances peuvent être utilisées seules ou en combinaison de deux d'entre elles ou plus.

Des exemples de tensioactifs cationiques englobent les acides aminés, les sels d'alklamine, les sels d'ammonium quaternaires, les sels de pyridium, etc.

Des exemples d'acides aminés englobent la lécithine de jaune d'oeuf ou de soja, ou un dérivé de lécithine tel que la lécithine hydrogénée, l'hydroxyde de lécithine, etc.

Les sels d'alkylamine sont des sels d'amine primaire ou secondaire comportant 3 à 22 atomes de carbone et d'un acide carboxylique comportant 1 à 22 atomes de carbone ou d'un acide minéral. Et des exemples de ceux-ci englobent le sel d'acétate de dodécylamine, le sel de chlorhydrate de dodécylamine, le sel de stéarate de dodécylaline, le sel de stéarate de diméthylamine, etc.

Les sels d'ammonium quaternaires sont des sels d'amine quaternaire comportant 3 à 22 atomes de carbone, et d'un acide carboxylique comportant 1 à 22 atomes de carbone ou d'un acide minéral. Des exemples de ceux-ci englobent le chlorure de stéaryltriméthylammonium, le chlorure de lauryltriméthylammonium, le chlorure de distéaryldiméthylammonium, le chlorure de benzalconium, le chlorure de benzétonium, le sel bromure de palme-alkyl(de 10 à 14 atomes de carbone)isoquinolinium, le sel chlorure de dodécylimidazonium.

Des exemples de sels de pyridinium englobent le chlorure de poly(N,N-diméthyl-3,5-méthylène-pipéridinium), le chlorure de cétylpyridinium, etc.

D'autres exemples de tensioactifs cationiques englobent les oxydes d'amine, tels que l'oxyde de dodécyldiméthylamine, un polymère cationique tel qu'un copolymère acide acrylique-acide β-N,N-diméthyl-N-éthylaminoacétique-vinylpyrrolidone, etc.

Des exemples de tensioactifs amphotères englobent les bétaines, les phosphobétaines, les sulfobétaines, les glycinebétaines, les bétaines d'imidazolium, les oxydes d'amine. Plus spécifiquement, des exemples de bétaines englobent l'acétate de dodécyldiméthylamino-bétaine, l'acétate de stéaryldiméthylamino-bétaine, l'acétate d'amidopropyldiméthylamino-bétaine d'acide décanique, etc. Des exemples de phosphobétaïnes englobent le 2-(diméthyldodécylammonio)propiophosphate, le 2-(diméthyldodécylammonio)-2-hydroxypropiophosphate, etc. Des exemples de sulfobétaines englobent le dodécyldiméthyléthosulfate d'ammonium, etc. Des exemples de glycinebétaïne englobent la dodécyldi(aminoéthyl)glycine, etc. Des exemples de bétaïne d'imidazolium englobent la 2-undécyl-N,N,N-(hydroxyéthylcarboxyméthyl)-2-imidazoline de sodium, l'hydroxyde de 2-coprah-2-imidazolinium, le sel de sodium de 1-carboxyéthoxy-2, la bétaine de 2-heptadécyl-N-carboxyméthyl-N-hydroxyéthylimidazolinium, etc.

Des exemples préférés des tensioactifs englobent un ester d'acide gras de saccharose tel que le béhénate de saccharose, le stéarate de saccharose, le palmitate de saccharose, le myristate de saccharose, le laurate de saccharose, l'éruciate de saccharose, l'oléate de saccharose, un ester d'acide gras de glycérine tel que le monostéarate de glycéryle et l'oléate de glycéryle, un ester d'acide gras de polyglycéryle tel que le polyglycérineester d'acide hydroxystéarique condensé, le polyglycérylester de ricinoléate condensé, la lécithine telel que la lécithine de jaune d'oeuf et la lécithine de soja, les dérivés de lécithine tels que la lécithine de soja hydrogénée et l'hydroxyde de lécithine. Ces substances sont faiblement irritantes et peuvent aussi être utilisées comme additifs alimentaires. Elles peuvent être utilisées seules ou en combinaison de deux d'entre elles ou plus.

La teneur en tensioactif est convenablement choisie en fonction des produits cosmétiques recherchés, de la quantité de mousse et du diamètre de la mousse, et elle s'échelonne habituellement de 0,1 à 20 % en poids, de préférence de 0,5 à 10 % en poids.

En plus des composants décrits ci-dessus, de l'eau pure, de l'eau de source chaude, de l'eau de mer profonde, des solvants organiques, des agents épaississants, des agents colorants, des agents de conservation de l'humidité, des astringents, des agents de blanchiment, des agents anti-UV, des agents anti-inflammatoires, des agents activant la peau (cellule), des agents carbonatés, des anti-oxydants, des agents antiseptiques et des bactéricides, des agents chélatants, des agents de prévention de la décoloration, des agents tampon, des parfums, etc., peuvent être correctement utilisés en fonction des utilisations des produits cosmétiques en mousse de sorte que l'effet de la présente invention ne soit pas altéré.

Des exemples des solvants organiques qui peuvent être utilisés dans la composition de la présente invention englobent l'éthanol, l'acétone, l'acétate d'éthyle, l'acétate de butyle, le 1,3-butylèneglycol, l'éthylèneglycol, le diéthylèneglycol, le triéthylèneglycol, le polyéthylèneglycol, le propylèneglycol, le dipropylèneglycol, la glycérine, le butanol, le propanol, etc.

Des exemples des polymères hydrophiles supérieurs et des agents épaississants englobent les polymères supérieurs à base de végétaux tels que la gomme arabique, la gomme adragante, la gomme galactam, la gomme de graine de caroube, la gomme guar, la gomme de karaya, la mousse perlée, la pectine, l'agar, les algues colloides, le fucoidan, la gomme tragant, la gomme de graine de coine, le galactomannan, etc., les polymères supérieurs à base de microbes tels que la gomme xanthane, le curdlan, la gomme gélifiante, le gel fucacé, le dextran, le succinoglucan, le pullulan, les polymères suppérieurs à base animale tels que le chitosan, la caséine, l'albumine, la gélatine, etc., les polymères supérieurs à base d'amidon tels que l'amidon, le carboxyméthylamidon, le méthylhydroxypropylamidon, etc. Les polymères supérieurs à base de cellulose tels que la méthylcellulose, l'éthylcellulose, la méthylhydroxypropylcellulose, la carboxyméthylcellulose, l'hydroxyméthylcellulose, l'hydroxypropyl-cellulose, la nitrocellulose, le sel de sodium de sulfate de cellulose, la carboxyméthylcellulose de sodium, la cellulose cristalline, la poudre de cellulose, les polymères supérieurs à base d'acide alginique tels que l'alginate de sodium, l'alginate de propylèneglycol, etc., les polymères supérieurs à base de vinyle tels que le polyvinylméthyléther, un polymère carboxyvinylique, un polymère carboxyvinylique modifié par un groupe alkyle, etc., les polymères supérieurs à base de polyoxyéthylène, les polymères supérieurs à base d'un copolymère polyoxyéthylène.polyoxypropylène, les polymères supérieurs à base acrylique tels que le poly (acrylate de sodium), le poly(acrylate d'éthyle), le polyacrylamide, etc., les polymères supérieurs minéraux solubles dans l'eau tels que la polyéthylèneimine, un polymère cationique, la bentonite, la laponite, l'hetorite, etc., un argile minéral modifié par un composé organique tel que l'argile montmorillonite de diméthylbenzyldodécylammonium, l'argile montmorillonite de diméthyldioctadécylammonium, etc. De plus, un agent filmogène tel que le poly(alcool vinylique), la polyvinylpyrrolidone, etc., est aussi compris. Un, deux ou plusieurs de ces matériaux peuvent être utilisés.

Des exemples de l'agent colorant englobent les colorants synthétiques organiques, les colorants naturels, les pigments, une poudre de polymères supérieurs.

Plus spécifiquement, des exemples des colorants synthétiques organiques englobent les colorants azoiques (yellow N° 5 et red N° 505, etc.), les colorants à base de xanthène (red N° 213 et red N° 230, etc.), les colorants à base de quinoléine (yellow N° 204, etc.), les colorants à base de triphényméthane (blue N° 1, etc.), les colorants à base d'anthraquinone (green N° 201, etc.), les colorants à base d'indigo, etc., les pigments de verni (red N° 202, red N° 208), red N° 228, red N° 226, blue N° 404, etc., et des exemples de colorants naturels englobent les carotènes, la carthamine, les cochinéales, etc.

Des exemples de pigments englobent un pigment de verni, un pigment organique, un pigment minéral tel qu'un pigment de couleur, un pigment blanc, un pigment diluant, un pigment nacré, un pigment à lustre métallique, un pigment de paillettes de verre, un pigment minéral enrobé de métal, un pigment de résine, des poudres de polymères supérieurs, un pigment fonctionnel, etc. Un ou plusieurs de ces pigments sont utilisés.

Il existe deux types de pigment de verni. L'un est un pigment dans lequel un colorant soluble dans l'eau est rendu insoluble dans l'eau sous forme de sels de calcium, etc. Des exemples de ceux-ci englobent red N° 202, red N° 204, red N° 206, red N° 207, red N° 208, red N° 220, etc. L'autre est le pigment dans lequel un colorant soluble dans l'eau est rendu insoluble dans l'eau avec du sulfate d'aluminium, du sulfate de zirconium, etc., et est adsorbé sur de l'alumine. Des exemples de ceux-ci englobent yellow N° 5, red N° 230, etc.

Le pigment organique est constitué d'une poudre colorée qui ne contient pas de groupe hydrophile dans sa structure moléculaire, et ne se dissout pas dans l'eau, une huile et des solvants, et le pouvoir colorant et la résistance à la lumière de celui-ci sont excellentes. Des exemples de celui-ci englobent red N° 228 de pigment azoique, red N° 226 de pigment à base d'indigo, blue N° 404 de pigment à base de phtalocyanine, etc.,

Des exemples de pigment minéral englobent les oxydes de fer ayant différentes couleurs, tels que l'oxyde de fer rouge, l'oxyde de fer jaune, l'oxyde de fer noir, etc., le bleu outre-manche, le bleu de milori, l'oxyde de chrome, l'hydroxyde de chrome, l'oxyde de magnésium, l'oxyde de cobalt, l'oxyde de cobalt-titane, le noir de carbone, le violet de manganèse, le violet de cobalt, etc.,

Le pigment blanc est utilisé pour colorer, recouvrir ou analogue, et des exemples de celui-ci englobent le dioxyde de titane et l'oxyde de zinc.

Le pigment diluant est utilisé pour maintenir les configuration des produits, contrôler la capacité d'étalement, l'adhérence et le lustre de ceux-ci et ajuster la teinte de couleur de ceux-ci (en tant qu'agent de dilution) plutôt que comme colorant, et des exemples de celui-ci englobent le pigment à base de mica, la muscovite, le mica synthétique, la phlogopite, le mica rouge, la biotite, le mica de lithium, etc., les argiles minérales telles que la séricite, le talc, le kaolin, la montmorillonite, une zéolite, etc., les poudres minérales synthétiques telles que le carbonate de magnésium, le carbonate de calcium, l'acide silicique, l'acide silicique anhydre, le silicate d'aluminium, le silicate de magnésium, le silicate de magnésium-aluminium, le silicate d'aluminium contenant du soufre, le silicate de calcium, le silicate de baryum, le silicate de strontium, l'oxyde d'aluminium, le sulfate de baryum, etc.,

Le pigment nacré est le pigment à utiliser pour donner un lustre perlé, les couleurs de l'arc-en-ciel et une sensation métallique aux produits, et des exemples de celui-ci englobent le dioxyde de titane recouvrant du mica, les paillettes d'écailles de poisson, l'oxychlorure de bismuth, etc. De plus, un pigment recouvert d'oxyde de fer au lieu du dioxyde de titane, le pigment portant une couche de couverture de titanate recouverte d'un autre pigment d'une couleur transparente différente, etc., sont utilisés.

Des exemples de pigment à lustre métallique englobent une poudre d'aluminium, une poudre de laiton, une poudre de cuivre, une poudre d'étain, une poussière d'or, une poudre d'argent, etc., et un pigment de poudre métallique coloré préparé par coloration de ces poudres métalliques.

Le pigment de paillettes de verre est préparé par revêtement de verre sous forme de paillettes par un métal, etc.

Le pigment minéral enrobé de métal est un pigment minéral qui est recouvert d'un métal et/ou d'un oxyde métallique par évaporation métallique, et des exemples de celui-ci englobent l'oxyde de fer recouvrant de l'aluminium, de l'oxyde de fer recouvrant du mica, de l'aluminium-manganèse recouvrant de l'oxyde de fer à base de mica, etc.

Un pigment de résine est constitué d'une paillette de résine préparée par coloration d'un film de résine et par découpage du film de résine coloré en paillettes, des exemples de pigment de résine englobent une poudre de film de polyester, un film stratifié poly(téréphalate d'éthylène)-aluminium-époxy, un film stratifié poly(téréphalate d'éthylène)-polyoléfine, le poly(méthacrylate de méthyle), un stratifié poly(téréphalate d'éthylène)-poly(méthacrylate de méthyle), une poudre de nylon, etc.,

Des exemples de pigment fonctionnel englobent le nitrure de bore, la phogopite d'or et de bore synthétique, un pigment photochromique, une poudre composite à grain fin, etc.

La forme de ces pigments de blanchiment n'est pas spécifiquement limitée, et elle peut être choisie arbitrairement parmi une forme granulaire, une forme de feuille, une forme de tige, etc., selon le but et le type de pigment à utiliser.

La taille du pigment n'est pas spécifiquement limitée, et elle peut être arbitrairement choisie selon le but et le type de pigment à utiliser. Dans le cas de la forme granulaire, un pigment ayant un diamètre moyen de particule s'échelonnant de 0,01 $\mu$m à 5 000 $\mu$m est habituellement utilisé et, dans le cas d'une forme en paillette ou une forme en tige, un pigment ayant un diamètre de particule plus long s'échelonnant de 0,1 $\mu$m à 5 000 $\mu$m est utilisé.

Des exemples d'humidifiant à utiliser dans les produits cosmétiques en mousse de la présente invention englobent l'eau de source simple alcaline, l'eau de mer profonde, les mucopolysaccharides tels que l'acide hyaluronique, le sulfate de chondroitine, le sulfate de dermatan, le sulfate d'héparan, l'héparine, le sulfate de kératan, etc., ou les sels de ceux-ci, les protéines telles que le collagène, l'élastine, la kératine, les dérivés de celles-ci, et les sels de celles-ci, les phospholipides dérivés du soja et des oeufs, les glycolipides, le céramide, la mucine, le miel, l'érythritol, le maltose, le maltitol, le xylitol, le xylose, le pentaérythritol, le fructose, la dextrine et les dérivés de ceux-ci, les saccharides tels que le mannitol, le sorbitol, l'inositol, le tréhalose, le glucose, etc., l'urée, l'asparagine, l'acide aspartique, l'alanine, l'arginine, l'isoleucine, l'orthinine, la glutamine, la glycine, l'acide glutamique, les dérivés de ceux-ci et les sels de ceux-ci, la cystéine, la cystine, la citrulline, la thréonine, la cérine, la tyrosine, le tryptophan, la théanine, la valine, l'histidine, l'hydroxylysine, l'hydroxyproline, le carbonate de pyrrolidone, les sels de celui-ci, les acides aminés tels que la proline, la phénylalanine, la méthionine, la lysine, les dérivés de ceux-ci et les sels de ceux-ci, le D-panthénol, les liquides extraits de végétaux. Des exemples de liquides extraits de végétaux englobent l'extrait d'avocat, l'huile d'amande, l'extrait de carouble, l'extrait de plante de riz, l'extrait de fraise, l'extrait d'anis, l'extrait de rose trémière rouge clair, l'extrait de coptise, l'huile d'olive, l'extrait de lamium album, le beurre de cacao, lextrait d'orge sauvage, l'extrait de lierre, l'extrait de sasa albo-marginata, l'extrait de gardénia, l'extrait de pamplemousse, l'extrait de geranium thumbergii, l'extrait de gentiane jaune éclatant, l'extrait de bardane, l'extrait de pivoine, l'extrait de sésame, l'extrait de cactus, l'extrait de saponaria officinalis, l'extrait de gingembre, l'extrait de racine de rehmannia, le beurre de mouton, l'extrait de spiraea, l'extrait de onidium officinale, l'extrait de guimauve, l'extrait de thymus vulgaris, l'extrait de camellia, l'extrait de blé, l'extrait de cordyceps sinensis, l'extrait de tormentila, l'extrait de houttuynia, l'extrait d'ophiopogon tuber, l'extrait de lupinus perennis, l'extrait d'hamamelis, l'extrait de menthe, l'extrait de mentha viridis, l'extrait de menthe opivrée, l'extrait de persil, l'extrait de rose, l'extrait de tournesol, l'extrait de hinoki, l'extrait d'éponge végétale, l'extrait de prune, l'extrait de genêt, l'huile de bourache, l'extrait de pivoine, l'huile de jojoba, les extraits de tilia miqueliana, l'extrait de houblon, l'extrait de pin, l'extrait de marronnier, l'huile de noix de macadamia, les extraits de coing, les extraits de grémil, l'huile de mousse de prairie, les extraits de mélisse, les extraits de farine de blé, les extraits de lis, les extraits de citron, les extraits d'agrume, l'extrait de lavadula vera, l'extrait de gertianas scabra, les extraits de sanguisorba, les extraits de pomme, etc. Des exemples de composants humidifiants englobent, en outre, un métabolite de levure, un extrait d'extraction de levure, un extrait de fermentation de riz, un extrait de son de riz fermenté, un extrait d'euglène, un extrait de fermentation lactique de lait cru et de lait écrémé, les dérivés de tréhalose de celui-ci, les alcools et les polyols constitués d'alcools naturels tels que l'éthanol, l'isopropanol, l'alcool laurylique, le cétanol, l'alcool stéarylique, l'alcool oléylique, l'alcool lanolinique, le cholestérol, le phytostérol, etc., et les alcools synthétiques tels que le 2-hexyldécanol, l'alcool isostéarylique, le 2-octyldodécanol, etc., l'oxyde d'éthylène, l'éthylèneglycol, le diéthylèneglycol, le triéthylèneglycol, l'éther monoéthylique d'éthylèneglycol, l'éther monobutylique d'éthylèneglycol, l'éther monométhylique de diéthylèneglycol, l'éther monoéthylique de diéthylèneglycol, le polyéthylèneglycol, l'oxyde de propylène, le propylèneglycol, le

polypropylèneglycol, le 1,3-butylèneglycol, la glycérine, le pentaérythritol, le sorbitol, le mannitol, etc. L'un de ces composants humidifiants ou deux ou plusieurs d'entre eux peuvent être arbitrairement choisis et mélangés. La quantité mélangée d'humidifiant dépend du type de celui-ci, mais elle s'chelonne habituellement de 0,5 à 20 %.

Des exemples d'astringents (composants) à utiliser dans les produits cosmétiques en mousse de la présente invention englobent le phénolsulfonate de zinc, le phénolsulfonate de sodium et les liquide d'extraits végétaux. Des exemples des liquides d'extraits végétaux englobent les extraits liquides extraits de végétaux tels que l'arnica, l'aubépine, la cinchonille, la sauge, la tilia miqueliana, le ginseng, le junipérus communis, le romarin, l'hypericum, l'arbre aux quarante écus, le mélise, l'ononis spinosa, le marronnier, le swertia japonica, l'ail, la camomille, le thym, la menthe, l'ortie, le poivre rouge, le gingembre, le houblon, le marron d'Inde, la Lavandula vera, la carotte, la moutarde en feuille, le cinnamomum casia, le pin, le cnidium officinale, le sureau, l'ostericum sieboldii, le scopolia, la pivoine, la myrica, l'houttuynia, l'os d'orysa sativa, la plaquemine amère, le calendula officinalis, le coquelicot, le gertiana scabra, le raisin, la glehnia, l'orange, le citron, l'iris, l'orange d'été du Japon, l'hamamélis, le trèfle doux, l'anis, le poivre du Japon, la pivoine, l'eucalyptus, l'artemisia vulgaris indica, l'isodon japonicus, le riz, la sophora angustifolia, le pain, le clou de girofle, la feuille de châtaigne, la racine de scutellaria, la sauge, les racines tubéreuses de polygonom multiflorum, le coptidis rhizoma, le phellodendri cortex, le scuttellaria baicalensis GEORGI, l'Houttuyniae herba, l'aurantii nobilis pericarpium, la carotte, la pivoine, le codonopsitis radix, le propolis, l'alisma rhizome, le tannin, le goudron de bois de bouleau, le gelée royale, l'extrait de levure, etc. L'une de ces substances, ou deux ou plusieurs de celles-ci peuvent être utilisées en combinaison. Habituellement, la quantité des astringents s'échelonne de 0,001 à 5 % en poids, de préférence de 0,01 à 3 % en poids, par rapport à la quantité totale de la composition cosmétique.

Des exemples des agents de blanchiment (composants) à utiliser dans les produits cosmétiques en mousse de la présente invention englobent un inhibiteur de tyrosinase, un antagoniste de l'endothéline, un inhibiteur d'$\alpha$-MSH, la glabridine, le glablène, la liquilitine, l'isoliquilotine, l'acide ellagique, les sels de l'acide ellagique, les dérivés de l'acide ellagique, l'acide kojique, les sels de l'acide kojique, les dérivés de l'acide kojique, l'arbutine, les sels d'arbutine, les dérivés d'arbutine, la cystéine, les sels de cystéine, les dérivés de cystéine, la vitamine C telle que l'acide ascorbique, l'ascorbate de sodium, le stéarate d'ascorbyle, le palmitate d'ascorbyle, le dipalmitate d'ascorbyle, l'ascorbyl-2-phosphate de magnésium, les sels de vitamine C, les dérivés de vitamine C, la glutathione, les sels de glutathione, les dérivés de glutathione, la résorcine, les sels de résorcine, les dérivés de résorcine, le rucinol, la néoagarobiose, l'oligosaccharide d'agarose, et les liquides extraits végétaux. Des exemples des liquides extraits de végétaux englboent l'extrait d'asperge, l'extrait d'althaea officinalis, l'extrait de bistorta major, l'extrait d'artemisiae capillaris flos, l'extrait de pois, l'extrait de fruit de rose de chien, l'extrait de racine de scutellaria, l'extrait d'ononis spinosa, l'extrait d'algues, l'extrait de firethorm, l'extrait de réglisse, l'extrait de mûre, l'extrait de racine de sophora, l'extrait de sucre brun, l'extrait de millettja triculate, l'extrait d'acanthopanacis cortex, l'extrait de germe de mais, l'extrait d'asiasari radix, l'extrait d'aubépine, l'extrait de nomame herba, l'extrait de pivoine, l'extrait de lis, l'extrait de fleur d'inule, l'extrait d'écorce de mûrier, l'extrait de soja, l'extrait de placenta, l'extrait d'aralia elata, l'extrait de thé, l'extrait d'angélica acutiloba, l'extrait de mélase, l'extrait de rosa polyantha, l'extrait d'ampelopsis japonica, l'extrait de grain de raisin, l'extrait de bouleau, l'extrait de fleur de manite, l'extrait de houblon, l'extrait de rosa rugosae flos, l'extrait de chaenomelis fructus, l'extrait de saxifraga, l'extrait de coicis semen, l'extrait de rakanta, etc. Une de ces substances peut être utilisées, ou deux ou plusieurs d'entre elles peuvent être arbitrairement utilisées. Habituellement, la teneur des agents de blanchiment s'échelonne de 0,01 à 10 %. Lorsque les extraits végétaux ou analogue sont utilisés sous forme d'un liquide extrait, la teneur ci-dessus est la quantité convertie en extrait solide séché.

Les agents anti-ultraviolets (composants) à utiliser dans les produits cosmétiques en mousse de la présente invention englobent les absorbants d'ultraviolets à base de composés organiques et les agents dispersants les rayons ultraviolets à base de composés minéraux. Des exemples d'absorbants d'ultraviolets englobent les absorbants d'ultraviolets à base d'acide para-aminobenzoique, les absorbants d'ultraviolets à base d'acide cinnamique, les absorbants d'ultraviolets à base d'acide salicylique, les absorbants d'ultraviolets à base de benzophénone, etc. Un ou plusieurs de ces absorbant sont mélangés. Des exemples des absorbants d'ultraviolets à base d'acide para-aminobenzoique englobent l'acide para-aminobenzoique, le para-aminobenzoate de glycéryle, le para-aminobenzoate d'éthyldihydropropyle, le para-aminobenzoate de para-diméthyle, le para-aminobenzoate d'octyl-para-méthyl, le para-aminobenzoate d'éthyle, le para-aminobenzoate d'isobutyle, etc., des exemples des absorbants d'ultraviolets à base d'acide cinnamique englobent le para-méthoxycinnamate d'isopropyle, l'ester d'acide diisopropylsinnamique, le méthoxycinnamate d'octyle, le di-para-méthoxycinnamate de d'acide éthylhexanoique de glycéryle etc., des exemples des absorbants d'ultraviolets à base d'acide salicylique englobent le salicylate d'homomenthyle, le salicylate d'octyle, le salicylate de phényle, la triéthanolamine d'acide salicylique, le salicylate d'amyle, le salicylate de benzile, le salicylate de p-tert-butylphényle, le salicylate d'éthylèneglycol, l'acide salicylique, etc., des exemples des absorbants d'ultraviolets à base de benzophénone englobent la dihydroxybenzophénone, la tétrahydroxybenzophénone, l'oxybenzone, l'acide oxybenzonesulfonique, l'hydroxyméthoxybenzophénone-sulfonate de sodium, la dihydroxydiméthoxybenzophénone, la 2-hydroxychlorobenzophénone, la dioxybenzophénone, le dihydroxydiméthoxybenzophénonedisulfonate de sodium, la 2-hydroxy-4-méthoxy-4'-méthylbenzophénone l'octabenzone, l'acide urocanique, l'urocanate d'éthyle, le 4-tert-4'-méthoxydibenzoylméthane, le

2-(2'-hydroxy-5'-méthylphényl)benzotriazole, l'acide anthranilique, etc. Des exemples des comopsés minéraux à utiliser comme agents dispersant les rayons ultraviolets englobent l'oxyde de titane, l'oxyde de zinc, l'oxyde de cérium, l'oxyde de zirconium, l'oxyde de fer, etc.

Des exemples d'agents anti-inflammatoires (composants) à utiliser dans les produits cosmétiques en mousse de la présente invention englobent l'oxyde de zinc, le soufre, les dérivés du soufre, l'acide glycyrrhizinique, les dérivés et les sels de l'acide glycyrrhizinique, tels que le glycyrrhigate dipotassique et le glycyrrhigate de monoammonium, l'acide glycyrrhétique, les dérivés et les sels de l'acide glycyrrhétique tels que l'acide β-glycyrrhétique, le glycyrrhétinate de stéaryle, le 3-succinyloxyglycyrrhétinate disodique, etc., l'acide tranexamique, le sulfate de chondroïtine, l'acide méfénamique, la phénylbutazone, l'indométacine, l'ibuptofen, le cétoprofen, l'allantoine, le quaiazulène et les dérivés et les sels de celui-ci, divers types de microbes, les extraits animaux, les extraits végétaux, etc.

Des exemples d'activateur de la peau (cellule) (composants) à utiliser dans les produits cosmétiques en mousse de la présente invention englobent l'acide désoxyribonucléique, les sels de l'acide désoxyribonucléique, les dérivés d'acide adénylique, tels que l'adénosinetriphosphate, l'adénosinephosphate, les sels de celui-ci, l'acide ribonucléique, les sels de celui-ci, l'AMP cyclique, le GMP cyclique, la flavine, l'adéninedinucléotide, la guanine, l'adénine, la cytosine, la thymine, la xanthine et les dérivés de celle-ci, la caféine, la théophylline et les sels de celle-ci, le rétinol, les dérivés de rétinol tels que le palmitate de rétinol, l'acétate de rétinol, etc., le rétinal, les dérivés de rétinal tels que le déshydrorétinal, etc., la carotène, la vitamine A telle que le caroténoïde, etc., la thiamine, les sels de thiamine tels que le chlorhydrate de thiamine, le sulfate de thiamine, etc., la riboflavine, les sels de riboflavine tels que l'acétate de riboflavine, etc., la pyridoxine, les sels de pyridoxine tels que le chlorhydrate de pyridoxine, le dioctanoate de pyridoxine, etc., le flavineadéninedinucléotide, la cyanocobalamine, les acides foliques, l'acide nicotinique, les dérivés de l'acide nicotinique tels que le nicotinamide, l'acide benzilnitotinique, etc., la vitamine B telle que la choline, etc., l'acide γ-linolénique et les dérivés de celui-ci, l'acide éicosapentaénoïque et les dérivés de celui-ci, l'estradiol et les dérivés et les sels de celui-ci, les acides organiques tels que l'acide glycolique, l'acide succinique, l'acide lactique, l'acide salicylique, etc., et les dérivés et les sels de ceux-ci.

Des exemples d'agents antibactériens (composants) à utiliser dans les produits cosmétiques en mousse de la présente invention englobent l'acide benzoique, le benzoate de sodium, l'acide carbolique, l'acide sorbique, le sorbate de potassium, l'ester d'acide para-oxybenzoïque, le para-chlorométacrésol, l'hexachlorophène, le chlorure de benzalkonium, le chlorure de chlorhexidine, le trichlorocarbanilide, le guaternum, le bis(2-pyridylthio-1-oxyde) de zinc, le phénoxyéthanol, le thianthol, l'isopropylméthylphénol, etc.

Des exemples d'antioxydants (composants) à utiliser dans les produits cosmétiques en mousse de la présente invention englobent la vitamine A tels que le rétinol, le déshydrorétinol, l'acétate de rétinyle, le palmitate de rétinyle, le rétinal, l'acide rétinoïque, une huile de vitamine A, les dérivés et les sels de celle-ci, les caroténoïdes tels que l'α-carotène, la β-carotène, la γ-carotène, la cryptoxanthine, l'astaxanthine, la fucoxanthine, etc., et les dérivés et les sels de ceux-ci, la vitamine B telle que la pyridoxine, le pyridoxal, l'ester pyridoxal-5-phosphate, la pyridoxamine, etc., et les dérivés et les sels de celle-ci, la vitamine C telle que l'acide ascorbique, l'ascorbate de sodium, le stéarate d'ascorbyle, le palmitate d'ascorbyle, le dipalmitate d'ascorbyle, le phosphate d'acide ascorbique de magnésium, etc., et les dérivés et les sels de celle-ci, la vitamine D telle que l'ergocalciférol, le cholecalciférol, le 1,2,5-dihydroxy-cholecalciférol, etc., les dérivés et les sels de celle-ci, la vitamine E telle que l'α-tocophérol, le β-tocophérol, le γ-tocophérol, le δ-tocophérol, l'α-tocotriénol, le β-tocotriénol, le γ-tocotriénol, le δ-tocotriénol, l'acétate de tocophérol, le nicotinate de tocophérol, etc., les dérivés et les sels de celle-ci, le tororoks (dérivés de la vitamine E solubles dans l'eau), les dérivés et les sels de ceux-ci, le dihydroxytoluène, le butylhydroxytoluène, le butylhydroxyanisol, le dibutylhydroxytoluène, l'acide α-lipoïque, l'acide déshydralipoïque, la glutathione, les dérivés et les sels de celle-ci, l'acide urique, l'acide érythorbique, les dérivés et les sels de celui-ci tels que l'érythorbate de sodium, etc., l'acide gallique, les dérivés et les sels de celui-ci tels que le gallate de propyle, etc., la rutine, les dérivés et les sels de celle-ci tels que l'α-glycosylrutine, etc., le tryptophane, les dérivés et les sels de celui-ci, l'histidine, les dérivés et les sels de celle-ci, les dérivés et les sels de cystéine tels que la N-acétylcystéine, la N-acétylhomocystéine, la N-octanoylcystéine, l'ester méthylique de N-acétylcystéine, etc., les dérivés et les sels de cystine tels que l'ester diméthylique de N,N'-diacétylcystine, l'ester diméthylique de N,N'-dioctanoylcystine, l'ester diméthylique de N,N'-dioctanoylhomocystine, etc., la carnosine, les dérivés et les sels de celle-ci, l'homocarnosine, les dérivés et les sels de celle-ci, l'ansérine, les dérivés et les sels de celle-ci, la calcinine, les dérivés et les sels de celle-ci, les dérivés et les sels de dipeptide ou de tripeptide contenant de l'histidine et/ou du triptophan et/ou de l'histamine, les flavonoïdes tels que la flavanone, la flavone, l'anthocyanine, l'anthocyanidine, le flavonol, le quelcitron, la myricétine, la phycétine, l'hamamelis tannin, la catéchine, l'épicatéchine, la gallocatéchine, l'épigallocatéchine, le gallate d'épicatéchine, le gallate d'épigallocatéchine, etc., l'acide tannique, l'acide cafféinique, l'acide férulique, l'acide protocatécuique, la chalcone, l'oryzanol, le carnot sole, le sesamole, la sésamine, la sésamoléine, la zingérone, la curcumine, la tétrahydrocurcumine, le chlobamide, le désoxychlobamide, le shoga-ol, la capsaicine, le vanillylamide, l'acide ellagique, le bromophénol, la flavoglassine, la mélanoidine, la riboflavine, l'ester butylique de riboflavine, le flavinemononucléotide, le flavineadéninenucléotide, l'ubiquinone, l'ubiquinol, le mannitol, la bilirubine, le cholestérol, l'ébsélène, le sélénométhionine, la cérulplasmine, la transferrine, la lactoferrine, l'albumine, la bilirubine, la superoxydedismutase, la catalase, la glutathioneperoxydase, la métallothionéine, l'O-phosphono-pyridoxylidènerhodamine, l'acide N-(2-hydroxybenzyl)aminé, les dérivés et les sels de celui-ci, l'acide N-(4-pyridoxylméthylène)aminé, les dérivés et les sels de celui-ci, qui sont décrits dans le brevet américain N˚ 5 594 012, etc. La teneur en antioxydants dépend des types de ceux-ci de façon à ne pas les déterminer de façon égale. Mais, habituellement, la teneur de ceux-ci s'échelonne de 0,01 à 10 %. Lorsque l'extrait végétal ou analogue est utilisé comme liquide extrait, la teneur décrite ci-dessus est exprimée en quantité convertie d'un extrait solide sec.

Des exemples de parfums (composants) à utiliser dans les produits cosmétiques en mousse de la présente invention englobent les parfums naturels et les parfums synthétiques. Des exemples de parfums naturels englobent les parfums végétaux naturels tels que l'huile de rose, l'huile de jasmin, l'huile de néroli, l'huile de lavande, l'huile de tuberose, l'huile de Ylang Ylang, l'huile de sauge sclarée, l'huile de clou de girofle, l'huile de menthe pouvrée, l'huile de géranium, l'huile de patchouli, l'huile de santal, l'huile de cannelle, l'huile de coriandre, l'huile de muscade, l'huile de pin, l'huile de vanille, l'huile de balsam Peru, l'huile de banane, l'huile de pomme, l'huile de fenouil, l'huile de graine de tonca, l'huile de poivre, l'huile de citron, l'huile d'orange, l'huile de bergamote, l'huile d'opopanax, l'huile de vétiver, l'huile d'iris, l'huile de chêne, l'huile d'anise, l'huile de bois de rose, etc., et les parfums animaux naturels tels que l'huile de vison, l'huile de civet, l'huile de ricin, l'huile d'ambre gris, etc. Des exemples de parfums synthétiques englobent les hydrocarbures tels que la limonène, le β-caryophylène, etc., les alcools tels que le cis-3-hexénol, le linalool, le farnésol, l'alcool β-phényléthylique, le géraniol, le citronellol, le terpinéol, le menthol, le santalol, le bacdanol, le puramanol, etc., les aldéhydes tels que le lyranol, le lilial, le 2,6-nonadiénal, le citral, l'aldéhyde d'α-hexylcinnamique, etc., les cétones telles que la β-ionone, la 1-carvone, la cyclopentadécanone, la damascone, la méthylionone, l'irone, l'IOS E-super, l'acétylcédrène, la muscone, est., les esters tels que l'acétate de benzyle, le dihydrojasmonate de méthyle, le jasmonate de méthyle, l'acétate de linalyle, le benzoate de benzyle, etc., les lactones telles que la γ-undécalactone, la jasminelactone, le cyclopentadécanolide, le brassylate d'éthylène, etc., les oxydes tels que le galactsolide, l'ambroxane, l'oxyde de rose, etc., les phénols tels que l'eugénol, etc., les composés contenant de l'azote tels que l'indole, etc., les acétales tels que le phénylacétaldéhyediméthylcétal, etc., les bases de Schiff tels que l'aurantiol, etc. Habituellemen,t plusieurs de ces parfums sont utilisés en combinaison sous forme d'un mélange de parfums selon les buts de ceux-ci.

Les teneurs en agents conservant l'humidité, en astringents, en agents de blanchiment, en agents anti-UV, en agents anti-inflammatoires, en agents activant la peau (cellule) et en antibiotiques ne sont pas spécifiquement limitées, mais ils peuvent être habituellement ajoutés dans une gamme de 0,01 à 20 %, par rapport à la quantité totale des produits cosmétiques.

En utilisant le polysaccharide selon la présente invention, on peut maintenir de façon stable la mousse contenue dans les produits cosmétiques en mousse sans les modifier. Lorsque la température, la pression, la vibration, un choc ou analogue est appliqué sur les produits cosmétiques en mousse pendant le transport et le stockage de ceux-ci, le volume de la mousse n'est par notablement réduite et la mousse ne fond pas avec les autres pour donner une mousse importante, de sorte que la qualité des produits cosmétiques en mousse n'est pas modifiée. De plus, la capacité d'étalement et la sensation légère lors de l'utilisation des produits cosmétiques en mousse ne sont pas non plus compromises.

Dans la suite, la présente invention sera expliquée en détail en référence à plusieurs exemples, mais la présente invention n'est pas limitée à ces

exemples.

[Polysaccharide utilisé dans les expériences]

(A-1 : Polysaccharide d'un produit d'un micro-organisme de la souche Alcaligenes latus B-16 (produit brut))

On dissout dans une eau à ions échangés, 40,0 g de glucose [Wako Pure Chemical Industries, Ltd., regent], 4,0 g d'hydrogénophosphate dipotassique [Wako Pure Chemical Industries, Ltd., regent], 2,0 g de dihydrogénophosphate de potassium [Wako Pure Chemical Industries, Ltd., regent], 0,1 g de chlorure de sodium [Wako Pure Chemical Industries, Ltd., regent], 0,2 g de sulfate de magnésium [Wako Pure Chemical Industries, Ltd., regent], 1,0 g de nitrate de potassium [Wako Pure Chemical Industries, Ltd., regent] et 1,5 g d'extrait de levure [OXOID Co., Ltd.], et on ajuste la solution aqueuse obtenue à un pH de 6,5 en utilisant de l'hydroxyde de sodium ou de l'acide sulfurique, de sorte que le volume total soit de 1 litre. On transfère 150 ml de la solution aqueuse obtenue dans un ballon conique de 500 ml et on la stérilise par passage à l'autoclave à 121˚C pendant 15 minutes. Ensuite, on abaisse la température de la solution à la température ambiante, et on inocule la souche Alcaligenes latus B-16 (FERM BP-2015) dans la solution dans le ballon. Et, on soumet la solution à une culture sous agitation à 30˚C pendant 6 jours (180 tours par minute). Après la culture, on y ajoute environ trois volumes d'alcool isopropylique et on les agite pour les mélanger. On filtre l'agglomérat précipité résultant, on le récupère et on le sèche sous pression réduite pour obtenir des polysaccharides d'un produit d'un micro-organisme de la souche Alcaligenes latus B-16 (A-1). Les polysaccharides obtenus comprennent un polysaccharide constitué de fucose, de glucose, d'acide glucuronique et de rhamnose dans un rapport molaire de 1:2:1:1, en tant que

composant principal, et un autre polysaccharide constitué de fucose et de mannose dans un rapport molaire de 1:1. Le rapport du premier polysaccharide au second polysaccharide est de 7:1 (rapport en poids). On hydrolyse les polysaccharides avec de l'acide sulfurique, et on analyse les monosaccharides résultants avec une chromatographie liquide haute pression (HPLC).

(A-2 : produit purifié de A-1 ci-dessus)

On prépare une solution aqueuse de 0, 5 % en poids des polysaccharides A-1, et on y ajoute une solution aqueuse d'hydroxyde de sodium jusqu'à un pH de 12. On traite la solution aqueuse obtenue en utilisant des colonnes de résine à ions échangés "DIAON HPA-75 (OH-) (marque de commerce)" (fabriquée par Nippon Rensui Co.) à 8 Ru ou moins, et on la filtre avec un auxiliaire de filtration "Radiolight RL700" et un filtre à membrane de 5 μm pour éliminer les protéines, les acides nucléiques et les microbes. Après avoir ajusté le liquide filtré à pH 7 avec de l'acide chlorhydrique dilué, on réduit la pression du liquide, et on concentre le liquide. Ensuite, on précipite les polysaccharides en utilisant de l'acétone, et on les lave avec dix volumes d'acétone, pour obtenir les polysaccharides (A-2) constitués de fucose, de glucose, d'acide glucuronique et de rhamnose dans un rapport molaire de 1:2:1:1, et ayant une masse moléculaire élevée de cinquante million.

[(B) Polysaccharides utilisés dans les exemples comparatifs]

- Gélatine : qualité réactif, fabriqué par Kanto Kaguku Co.
- Gomme xanthane : "Kersan T (marque de commerce)", fabriquée par Sansho Co., Ltd.
- Méthylcellulose : "Metrose AM (marque de commerce)" fabriquée par Shin-etsu Chemical Co. Ltd.
- Gomme agar : qualité réactif, fabriquée par Kanto Kaguku Co.
- Gomme gellan : "KELCOGEL LT100 (marque de commerce)", fabriquée par Sansho Co., Ltd.

[(C) Tensioactif]

- Monostéarate de glycérine : "Leodor MS-165 (marque de commerce)", fabriqué par Kao Corporation.
- Ester d'acide monooléique de sorbitan polyoxyéthoxylé (addition de 15 moles), fabriqué par Kao Corporation.
- Lecithine hydrogénée : "Basis LS-60RH (marque de commerce)", fabriquée par Nisshin Oil Co.
- Ester d'acide gras de saccharose : "L-160 (marque de commerce)", fabriqué par Dai-Ichi Kogyo Seiyaku Co., Ltd.
- Ester d'acide gras de polyglycérine : "O-15D (marque de commerce)", fabriqué par Mitsubishi Chemical Co., Ltd.
- Acide stéarique polyéthoxylé (addition de 40 moles) : fabriqué par Nikko Chemicals Co., Ltd.
- Oléyléther polyéthoxylé (addition de 15 moles) : fabriqué par Nikko Chemicals Co., Ltd.
- Huile de ricin polyéthoxylée (addition de 20 moles) : fabriquée par Nikko Chemicals Co., Ltd.
- Dioxyde de titane superfin : "TTO-D-1 (marque de commerce)", fabriqué par Ishihara Sangyo Kaisha, Ltd.
- Oxyde de zinc : diamètre moyen de particule de 0,2 à 0,6 μm, fabriqué par Hakushi Chemical Industries Co.
- Talc : "Victory Light SK-1 (marque de commerce)", fabriqué par Shokosan Laboratories.
- Mica : "MICA Y-3000SC (marque de commerce)", fabriqué par Yamagushi-Mica Co., Ltd.
- Oxyde de fer rouge : "140ED (marque de commerce)", fabriqué par Toda Kogyo Corp.
- L'oxyde de fer jaune, l'oxyde de fer rouge et l'oxyde de fer noir utilisés sont fabriqués par KISHI KASEI Co., Ltd.
- Le propylèneglycol, le 1,3-butylèneglycol, la glycérine, le sorbitol à 70 %, le parabène, le p-hydroxybenzoate de méthyle et l'ascorbate-phosphate de magnésium utilisés sont des réactifs fabriqués par Kanto Kagaku Co.
- La paraffine liquide, le squalane, la vaseline, l'acide stéarique, le cétanol, le myristate d'isopropyle, l'alcool béhénylique, l'isooctanoate de cétyle, l'huile de ricin hydrogénée polyéthoxylée (addition de 20 moles), le polyéthylèneglycol 1500, le poly(alcool vinylique), red N° 202, red N° 201, red N° 223 et le polyoxyéthanol utilisés sont fabriqués par Nikko Chemicals Co., Ltd.

[Préparation de produits cosmétiques en mousse]

(Exemple 1 : crème retenant l'humidité 1 <type crème H/E>)

On place dans un récipient de 500 ml les composants N° 1 à 9, à savoir 2,00 g de stéarate polyéthoxylé (addition de 40 moles), 0,50 g de monostéarate de glycérine, 5,00 g d'acide stéarique, 5,00 g d'alcool bénénylique,

10,00 g de squalane, 2,00 g de vaseline, 5,00 g d'isooctanate de cétyle, 0,10 g de p-hydroxybenzoate de méthyle et 5,00 g de 1,3-butylèneglycol, on les chauffe à 70˚C et on les mélange pour préparer un mélange non aqueux 1. De même, on place dans un récipient de 200 ml les composants N˚ 10 à 12, à savoir 64,89 g d'eau pure, 0,01 g de polysaccharide A-1 et 0,50 g d'extraits de graines de coix lachryma jobi, et on les mélange intimement avec un mélangeur à pales et on les chauffe à 70˚C pour préparer un mélange aqueux 2. On ajoute le mélange aqueux 2 au mélange non aqueux 1 tout en agitant avec un autohomogénisateur TK de type M (marque de commerce, fabriqué par Tokushu Kogyo Co., Ltd.) à 6 000 tours par minute pour préparer une émulsion. On transfère l'émulsion obtenue dans un ballon de mesure de 100 ml, et on mesure le poids de celle-ci. Ensuite, on fouette (forme une mousse) l'émulsion obtenue en mélangeant avec un mélangeur manuel (fabriqué par Kaijirushi Co.) pendant 5 minutes. Par la suite, on refroidit le mélange en l'agitant avec un agitateur à pales jusqu'à la température ambiante, et on obtient une crème retenant l'humidité 1 ayant une teneur en mousse de 52 % en poids (appelé "%" dans la suite).

| (N˚) | (COMPOSANTS DE MELANGE) (% en poids) | |
|---|---|---|
| 1. | stéarate polyéthoxylé (addition de 40 moles) | 2,00 |
| 2. | monostéarate de glycérine | 0,50 |
| 3. | acide stéarique | 5,00 |
| 4. | alcool béhénylique | 5,00 |
| 5. | squalane | 10,00 |
| 6. | vaseline | 2,00 |
| 7. | isooctanate de cétyle | 5,00 |
| 8. | p-hydroxybenzoate | 0,10 |
| 9. | 1,3-butylèneglycol | 5,00 |
| 10. | polysaccharide (A-1) | 0,01 |
| 11. | extrait de graines de coix lachryma kobi | 0,50 |
| 12. | eau pure | 64,89 |

(Exemple 2 : crème retenant l'humidité 2 <type crème H/E>)

On obtient une crème retenant l'humidité 2 ayant une teneur en mousse de 53 % par le même procédé que dans l'exemple 1, sauf que l'on remplace "64,89 g d'eau pure et 0,01 de polysaccharide A-1" dans la crème retenant l'humidité 1 de l'exemple 1 par "64,87 g d'eau pure et 0,03 de polysaccharide A-1".

(Exemple 3 : crème retenant l'humidité 3 <type crème H/E>)

On obtient une crème retenant l'humidité 3 ayant une teneur en mousse de 54 % par le même procédé que dans l'exemple 1, sauf que l'on remplace "64,89 g d'eau pure et 0,01 de polysaccharide A-1" dans la crème retenant l'humidité 1 de l'exemple 1 par "64,85 g d'eau pure et 0,05 de polysaccharide A-1".

(Exemple 4 : crème retenant l'humidité 4 <type crème H/E>)

On obtient une crème retenant l'humidité 4 ayant une teneur en mousse de 53 % par le même procédé que dans l'exemple 1, sauf que l'on remplace "64,89 g d'eau pure et 0,01 de polysaccharide A-1" dans la crème retenant l'humidité 1 de l'exemple 1 par "64,80 g d'eau pure et 0,10 de polysaccharide A-1"

(Exemple 5 : crème retenant l'humidité 5 <type crème H/E>)

On obtient une crème retenant l'humidité 5 ayant une teneur en mousse de 56 % par le même procédé que dans l'exemple 1, sauf que l'on remplace "64,89 g d'eau pure et 0,01 de polysaccharide A-1" dans la crème retenant l'humidité 1 de l'exemple 1 par "64,60 g d'eau pure et 0,30 de polysaccharide A-1".

(Exemple 6 : crème retenant l'humidité 6 <type crème H/E>)

On obtient une crème retenant l'humidité 6 ayant une teneur en mousse de 59 % par le même procédé que dans l'exemple 1, sauf que l'on remplace "64,89 g d'eau pure et 0,01 de polysaccharide A-1" dans la crème retenant l'humidité 1 de l'exemple 1 par "64,40 g d'eau pure et 0,50 de polysaccharide A-1". On prépare des produits cosmétiques en mousse ayant divers types de compositions de mélange par des procédés similaires de la manière suivante.

(Exemple 7 : crème de blanchiment 1 <type crème H/E>)

On obtient une crème de blanchiment 1 ayant une teneur en mousse de 56 % avec les rapports de mélange suivants.

| (N°) | (COMPOSANTS DE MELANGE) | (% en poids) |
|---|---|---|
| 1. | propylèneglycol | 5,00 |
| 2. | 1,3-butylèneglycol | 5,00 |
| 3. | glycérine | 5,00 |
| 4. | ester de saccharose | 0,50 |
| 5. | monostéarate de glycérine | 0,50 |
| 6. | p-hydroxybenzoate | 0,10 |
| 7. | squalane | 8,00 |
| 8. | acide stéarique | 4,00 |
| 9. | alcool béhénylique | 4,00 |
| 10. | cétanol | 4,00 |
| 11. | huile de diméthylsilicone | 2,00 |
| 12. | polysaccharide (A-1) | 0,01 |
| 13. | ascorbate-phosphate de magnésium | 3,00 |
| 14. | acide citrique | 1,00 |
| 15. | hydroxyde de sodium (neutralisation du pH) | quantité appropriée |
| 16. | eau pure | complément |

(Exemple 8 : crème solaire 1 <type crème H/E>)

On prépare une crème solaire avec les rapports de mélange suivants par un procédé similaire à celui de l'exemple 1. On mélange intimement à 70°C les composants N° 12 et 15, chacun en une quantité prédéterminée. On chauffe à 70°C les composants N° 1 à 6, chacun en une quantité prédéterminée, et on les mélange intimement, et on chauffe à 70°C les composants N° 7 à 11, chacun en une quantité prédéterminée, et on les mélange intimement. On ajoute successivement le mélange résultant des composants N° 1 à 6 et le mélange résultant des composants N° 7 à 11 au mélange résultant des composants 12 et 15 tout en les agitant avec un homomélangeur, afin de préparer une émulsion. Ensuite, on ajoute successivement des particules superfines de dioxyde de titane en tant que composant minéral N° 13 et de l'oxyde de zinc en tant que composant minéral N° 14, on les agite et on les refroidit tout en fouettant l'émulsion avec un mélangeur manuel. On obtient ainsi une crème solaire ayant une teneur en mousse de 47 %.

| (N°) | (COMPOSANTS DE MELANGE) | (% en poids) |
|---|---|---|
| 1. | propylèneglycol | 5,00 |
| 2. | 1,3-butylèneglycol | 5,00 |
| 3. | glycérine | 5,00 |
| 4. | ester de saccharose | 1,00 |
| 5. | lécthine hydrogénée | 0,30 |
| 6. | p-hydroxybenzoate | 0,10 |
| 7. | squalane | 3,00 |
| 8. | myristate d'isopropyle | 6,00 |
| 9. | acide stéarique | 4,00 |
| 10. | alcool béhénylique | 4,00 |
| 11. | cétanol | 4,00 |
| 12. | polysaccharide (A-2) | 0,10 |
| 13. | dioxyde de titane superfin | 5,00 |
| 14. | oxyde de zinc | 3,00 |
| 15. | eau pure | 54,50 |

(Exemple 9 : crème de maquillage pour les yeux 1 <type crème H/E>)

On obtient une crème de maquillage pour les yeux ayant une teneur en mousse de 40 % et les rapports de mélange suivants par un procédé similaire à celui de l'exemple 8.

| (N˚). | (COMPOSANTS DE MELANGE) | (% en poids) |
|---|---|---|
| 1. | propylèneglycol | 4,00 |
| 2. | 1,3-butylèneglycol | 4,00 |
| 3. | glycérine | 4,00 |
| 4. | ester de saccharose | 1,00 |
| 5. | monostéarate de glycérine | 1,00 |
| 6. | squalane | 6,00 |
| 7. | vaseline | 2,00 |
| 8. | acide stéarique | 2,00 |
| 9. | cétanol | 3,00 |
| 10. | myristate d'isopropyle | 6,00 |
| 11. | mica | 1,00 |
| 12. | dioxyde de titane superfin | 2,00 |
| 13. | oxyde de fer rouge | 0,20 |
| 14. | red N˚ 202 | 0,30 |
| 15. | p-hydroxybenzoate de méthyle | 0,10 |
| 16. | polysaccharide (A-2) | 0,10 |
| 17. | eau pure | 63,30 |

(Exemple 10 : crème de fond de teint 1 <type crème H/E>)

On obtient une crème de fond de teint ayant une teneur en mousse de 36 % et les rapports de mélange suivants par un procédé similaire à celui de l'exemple 8.

| (N˚) | (COMPOSANTS DE MELANGE) | (% en poids) |
|---|---|---|
| 1. | propylèneglycol | 3,00 |
| 2. | 1,3-butylèneglycol | 12,00 |
| 3. | glycérine | 3,00 |
| 4. | ester d'acide gras de saccharose | 1,00 |
| 5. | copolymère de méthylpolysiloxane polyéthoxylé et polypropoxylé | 0,50 |
| 6. | paraffine liquide | 5,00 |
| 7. | talc | 6,00 |
| 8. | dioxyde de titane superfin | 8,00 |
| 9. | oxyde de fer jaune | 1,00 |
| 10. | oxyde de fer rouge | 0,20 |
| 11. | oxyde de fer noir | 0,05 |
| 12. | p-hydroxybenzoate de méthyle | 0,10 |
| 13. | polysaccharide (A-2) | 0,10 |
| 14. | parfum | quantité appropriée |
| 15. | eau pure | complément |

(Exemple 11 : Rouge pour les joues 1 <type crème H/E>)

On obtient du rouge pour les joues ayant une teneur en mousse de 46 % et les rapports de mélange suivants par un procédé similaire à celui de l'exemple 8.

| (N˚) | (COMPOSANTS DE MELANGE) | (% en poids) |
|---|---|---|
| 1. | propylèneglycol | 4,00 |

(suite)

| (N°) | (COMPOSANTS DE MELANGE) | (% en poids) |
|---|---|---|
| 2. | 1,3-butylèneglycol | 4,00 |
| 3. | glycérine | 4,00 |
| 4. | ester d'acide gras de saccharose | 1,00 |
| 5. | monostéarate de glycérine | 1,00 |
| 6. | squalane | 6,00 |
| 7. | vaseline | 2,00 |
| 8. | acide stéarique | 2,00 |
| 9. | cétanol | 3,00 |
| 10. | myristate d'isopropyle | 6,00 |
| 11. | kaolin (qualité réactif, fabriqué | |
| par | Kanto Kagaku Co.) | 8,00 |
| 12. | dioxyde de titane (qualité réactif, fabriqué par Kanto Kagaku Co.) | 2,00 |
| 13. | oxyde de fer rouge | 0,20 |
| 14. | red N° 202 | 0,30 |
| 15. | p-hydroxybenzoate de méthyle | 0,10 |
| 16. | polysaccharide (A-1) | 0,10 |
| 17. | eau pure | 56,30 |

(Exemple 12 : rouge à lèvre 1 <type crème H/E>)

Après avoir préparé une émulsion à partir des composants N° 1 à 12 dans les rapports de mélange suivants par un procédé similaire à celui de l'exemple 8, on ajoute successivement les composants de mélange N° 13 à 17 tout en les fouettant avec un mélangeur manuel. On obtient ainsi un rouge à lèvre ayant une teneur en mousse de 43 %.

| (N°) | (COMPOSANTS DE, MELANGE) | (% en poids) |
|---|---|---|
| 1. | propylèneglycol | 4,00 |
| 2. | 1,3-butylèneglycol | 4,00 |
| 3. | glycérine | 4,00 |
| 4. | ester d'acide gras de saccharose (C-4) | 1,00 |
| 5. | lécithine hydrogénée (C-3) | 0,30 |
| 6. | squalane | 6,00 |
| 7. | vaseline | 2,00 |
| 8. | acide stéarique | 2,00 |
| 9. | cétanol | 3,00 |
| 10. | paraffine liquide (qualité réactif, fabriqué par Kanto Kagaku Co.) | 6,00 |
| 11. | paraffine solide (qualité réactif, fabriqué par Kanto Kagaku Co.) | 6,00 |
| 12. | dioxyde de titane (qualité réactif, fabriqué par Kanto Kagaku Co.) | 4,00 |
| 13. | red N° 201 | 0,50 |
| 14. | red N° 202 | 2,00 |
| 15. | red N° 223 | 0,10 |
| 16. | p-hydroxybenzoate de méthyle | 0,10 |
| 17. | polysaccharide (A-1) | 0,10 |
| 18. | eau pure | 54,90 |

(Exemple 13 : crème de nettoyage 1 <type crème H/E>)

On obtient une crème de nettoyage ayant une teneur en mousse de 52 % et les rapports de mélange suivants par un procédé similaire à celui de l'exemple 1.

| (N°) | (COMPOSANTS DE MELANGE) | (% en poids) |
|---|---|---|
| 1. | stéarate polyéthoxylé (addition de 40 moles) | 3,00 |
| 2. | monostéarate de glycérine | 2,50 |
| 3. | acide stéarique | 2,00 |
| 4. | cétanol | 3,00 |
| 5. | paraffine liquide | 20,00 |
| 6. | vaseline | 5,00 |
| 7. | myristate d'isopropyle | 5,00 |
| 8. | méthylphénylpolysiloxane | 5,00 |
| 9. | p-hydroxybenzoate | 0,10 |
| 10. | propylèneglycol | 5,00 |
| 11. | polysaccharide (A-1) | 0,10 |
| 12. | hydroxyde de potassium | quantité appropriée |
| 13. | eau pure | complément |

(Exemple 14 : crème capillaire 1 <type crème H/E>)

On obtient une crème capillaire ayant une teneur en mousse de 38 % et les rapports de mélange suivants par un procédé similaire à celui de l'exemple 1.

| (N°) | (COMPOSANTS DE MELANGE) | (% en poids) |
|---|---|---|
| 1. | propylèneglycol | 6,00 |
| 2. | 1,3-butylèneglycol | 10,00 |
| 3. | glycérine | 8,00 |
| 4. | huile de ricin hydrogénée polyéthoxylée (addition de 20 moles) | 2,00 |
| 5. | trioctanate de cétyle | 2,00 |
| 6. | vaseline | 4,00 |
| 7. | lécithine hydrogénée | 1,00 |
| 8. | phénoxyéthanol | 0,10 |
| 9. | parfum | quantité appropriée |
| 10. | polysaccharide (A-1) | 0,30 |
| 11. | eau pure | complément |

(Exemple 15 : gel de nettoyage 1 <type gel aqueux>)

On place dans un récipient les composants N° 1 à 4, 6 et 7, on les chauffe à 70°C sous agitation avec une pale et on les mélange pour obtenir une solution homogène. Après avoir arrêté le chauffage, on fait barboter de l'air dans le mélange à un débit de 1 ml/s dans le fond du récipient en utilisant une pompe à air à l'aide d'un tuyau de silicone ayant un diamètre de 5 mm (diamètre intérieur de 2,5 mm) tout en agitant à 200 tours par minute, puis le refroidit sous agitation pour obtenir une émulsion en mousse. Après avoir refroidi l'émulsion en mousse à la température ambiante et avoir ajouté une quantité appropriée de parfum N° 5, on soumet l'émulsion à un traitement au fouet (barbotage) en agitant avec un mélangeur manuel (fabriqué par Kaijirusi Co.) pendant 5 minutes, afin d'obtenir un gel de nettoyage 1 ayant une teneur en mousse de 52 %.

| (N°) | (COMPOSANTS DE MELANGE) | (% en poids) |
|---|---|---|
| 1. | propylèneglycol | 5,00 |
| 2. | dipropylèneglycol | 10,00 |
| 3. | lécithine hydrogénée | 2,50 |
| 4. | monooléate de sorbitan polyéthoxylé (addition de 15 moles) | 1,00 |
| 5. | parfum | 0,02 |
| 6. | polysaccharide (A-1) | 0,10. |
| 7. | eau pure | 81,38 |

(Exemple 16 : gel retenant l'humidité 1 <type gel aqueux>)

On prépare une émulsion avec les rapports de mélange suivants par un procédé similaire à celui de l'exemple 1, et on obtient un gel retenant l'humidité ayant une teneur en mousse de 48 %.

| (N°) | (COMPOSANTS DE MELANGE) | (% en poids) |
|---|---|---|
| 1. | dipropylèneglycol | 5,00 |
| 2. | polyéthylèneglycol 1500 | 10,00 |
| 3. | oléyléther polyéthoxylé (addition de 15 moles) | 1,00 |
| 4. | phénoxyéthanol | 0,10 |
| 5. | polysaccharide (A-1) | 0,30 |
| 6. | parfum | quantité appropriée |
| 7. | eau pure | complément |

(Exemple 17 : gel de plâtre 1 <type gel aqueux>)

On prépare une émulsion avec les rapports de mélange suivants par un procédé similaire à celui de l'exemple 1, et on obtient un gel de plâtre 1 ayant une teneur en mousse de 53 %.

| (N°) | (COMPOSANTS DE MELANGE) | (% en poids) |
|---|---|---|
| 1. | poly(alcool vinylique) | 10,00 |
| 2. | sorbitol à 70 % | 6,00 |
| 3. | 1,3-butylèneglycol | 4,00 |
| 4. | glycérine | 4,00 |
| 5. | éthanol | 8,00 |
| 6. | polysaccharide (A-1) | 0,30 |
| 7. | parfum | quantité appropriée |
| 8. | eau pure | complément |

(Exemple 18 : lotion <type soluble dans les composants huileux>)

On place dans un récipient les composants N° 11 et 12, on y ajoute un mélange des composants N° 1 à 10, tout en agitant avec un autohomogénisateur TK de type M (marque de commerce, fabriqué par Tokushu Kogyo Co., Ltd.) à 6 000 tours par minute, puis on insuffle de l'air par le fond du récipient à air à un débit de 1 ml/s en utilisant une pompe à air à l'aide d'un tuyau de silicone ayant un diamètre de 5 mm (diamètre interne de 2,5 mm) pour faire barboter le mélange. On obtient ainsi une lotion ayant une teneur en mousse de 5 %.

| (N°) | (COMPOSANTS DE MELANGE) | (% en poids) |
|---|---|---|
| 1. | éthanol | 6,00 |
| 2. | 1,3-butylèneglycol | 3,00 |
| 3. | glycérine | 5,00 |
| 4. | monooléate de sorbitan polyéthoxylé (addition de 20 moles) | 0,50 |
| 5. | huile de ricin hydrogénée polyéthoxylée (addition de 80 moles) | 0,50 |
| 6. | éther d'alcool laurylique polyéthoxylé (addition de 15 moles) | 0,50 |
| 7. | alcool oléylique | 0,10 |
| 8. | acétate de tocophérol | 0,05 |
| 9. | parfum | 0,05 |
| 10. | méthylparabène | 0,10 |
| 11. | polysaccharide (A-1) | 0,05 |
| 12. | eau pure | 84,15 |

(Exemple 19 : poudre liquide <type suspension aqueuse>)

On obtient une poudre liquide ayant une teneur en mousse de 16 % et les rapports de mélange suivants par un procédé similaire à celui de l'exemple 18.

| (N˚) | (COMPOSANTS DE MELANGE) | (% en poids) |
|---|---|---|
| 1. | éthanol | 14,00 |
| 2. | glycérine | 4,00 |
| 3. | 1,3-butylèneglycol | 2,00 |
| 4. | oxyde de fer jaune | 1,00 |
| 5. | oxyde de fer rouge | 0,05 |
| 6. | oxyde de fer noir | 0,05 |
| 7. | oxyde de zinc | 0,50 |
| 8. | kaolin | 2,00 |
| 9. | polysaccharide B-16 (A-2) | 0,05 |
| 10. | camphre | 0,15 |
| 11. | ascorbate-phosphate de magnésium | 3,00 |
| 12. | acide citrique | 1,00 |
| 13. | hydroxyde de sodium (contrôle du pH) | quantité appropriée |
| 14. | méthylparabène | 0,10 |
| 15. | eau de mer profonde | complément |

(Exemple 20 : lotion de type lait <type émulsion H/E>)

On obtient une lotion de type lait ayant une teneur en mousse de 34 % et les rapports de mélange suivants par un procédé similaire à celui de l'exemple 1.

| (N˚) | (COMPOSANTS DE MELANGE) | (% en poids) |
|---|---|---|
| 1. | diméthylpolysiloxane | 4,00 |
| 2. | squalane | 6,00 |
| 3. | alcool cétylique | 0,50 |
| 4. | acétate de tocophérol | 0,05 |
| 5. | extrait de feuille de sakvua iffucabalts (sauge) | 0,10 |
| 6. | extrait de fleur de centaurea cyanus | 0,10 |
| 7. | extrait de saxifraga saementosa | 0,10 |
| 8. | extrait de mogwort | 0,10 |
| 9. | 1,3-butylèneglycol | 4,00 |
| 10. | glycérine | 4,00 |
| 11. | éthanol | 6,00 |
| 12. | ester d'acide laurique de saccharose | 1,00 |
| 13. | méthylparabène | 0,10 |
| 14. | polysaccharide B-16 (A-2) | 0,06 |
| 15. | eau pure | 73,89 |

(Exemple 21 : lotion <type solution aqueuse>)

On obtient une lotion ayant une teneur en mousse de 28 % et les rapports de mélange suivants par un procédé similaire à celui de l'exemple 1.

| (N˚) | (COMPOSANTS DE MELANGE) | (% en poids) |
|---|---|---|
| 1. | éthanol | 15,00 |
| 2. | 1,3-butylèneglycol | 6,00 |
| 3. | glycérine | 6,00 |

(suite)

| | | |
|---|---|---|
| 4. | acide citrique | 0,01 |
| 5. | citrate de sodium | 0,10 |
| 6. | arbutine | 0,50 |
| 7. | extrait de fruit d'actinidia chinensis (kiwi) | 0,50 |
| 8. | huile de ricin hydrogénée polyéthoxylée (addition de 60 moles) | 0,50 |
| 9. | méthylparabène | 0,10 |
| 10. | polysaccharide B-16 (A-1) | 0,10 |
| 11. | eau pure | 71,19 |

(Exemple 22 : lotion en gel <type gel aqueux>)

On obtient une ltion en gel ayant une teneur en mousse de 37 % et les rapports de mélange suivants par un procédé similaire à celui de l'exemple 1.

| (N°) | (COMPOSANTS DE MELANGE) | (% en poids) |
|---|---|---|
| 1. | éthanol | 8,00 |
| 2. | 1,3-butylèneglycol | 6,00 |
| 3. | glycérine | 4,00 |
| 4. | ascorbate-phosphate de magnésium | 3,00 |
| 5. | acide citrique | 0,50 |
| 6. | hydroxyde de sodium (contrôle du pH) | quantité appropriée |
| 7. | extrait de fruit d'actinidia chinensis (kiwi) | 0,50 |
| 8. | huile de ricin hydrogénée polyéthoxylée (addition de 60 moles) | 0,50 |
| 9. | méthylparabène | 0,10 |
| 10. | polysaccharide B-16 (A-2) | 0,15 |
| 11. | eau pure | complément |

(Exemple 23 : crème de massage <type crème E/H>)

On obtient une crème de massage ayant une teneur en mousse de 32 % et les rapports de mélange suivants par un procédé similaire à celui de l'exemple 1.

| (N°) | (COMPOSANTS DE MELANGE) | (% en poids) |
|---|---|---|
| 1. | paraffine solide | 2,00 |
| 2. | cire microcristalline | 1,00 |
| 3. | cire de carnauba | 2,00 |
| 4. | vaseline | 3,00 |
| 5. | squalane | 20,00 |
| 6. | isocyanate de cétyle | 5,00 |
| 7. | adipate d'hexadécyle | 3,00 |
| 8. | copolymère polyoxyéthylène-méthylpolysiloxane | 2,00 |
| 9. | butylparabène | 0,20 |
| 10. | propylèneglycol | 6,00 |
| 11. | glycérine | 8,00 |
| 12. | triisostéarate de glycérol polyéthoxylé (addition de 20 moles) | 0,30 |
| 13. | huile de ricin hydrogénée polyéthoxylée (addition de 60 moles) | 1,50 |
| 14. | polysaccharide B-16 (A-2) | 0,10 |
| 15. | phénoxyéthanol | 0,20 |
| 16. | parfum | 0,01 |
| 17. | extrait de feuille de salvia officinalts (sauge) | 0,05 |
| 18. | extrait de rose (rosa multiflora) | 0,01 |

(suite)

| (N˚) | (COMPOSANTS DE MELANGE) | (% en poids) |
|---|---|---|
| 19. | extrait de chamomilla recutita (matricarta) | 0,05 |
| 20. | eau pure | 45,58 |

(Exemple 24 : huile de massage <type gel E/H>)

On obtient un gel de massage ayant une teneur en mousse de 42 % et les rapports de mélange suivants par un procédé similaire à celui de l'exemple 1.

| (N˚) | (COMPOSANTS DE MELANGE) | (% en poids) |
|---|---|---|
| 1. | paraffine liquide | 10,00 |
| 2. | squalane | 10,00 |
| 3. | huile d'avocat | 12,00 |
| 4. | extrait de racine de daucus carota sativa (carotte) | 8,00 |
| 5. | tri-2-éthylhexaneester de glycérol | 20,00 |
| 6. | butylparabène | 0,20 |
| 7. | acétate de tocophérol | 0,10 |
| 8. | propylèneglycol | 8,00 |
| 9. | glycérine | 5,00 |
| 10. | sorbitol | 6,00 |
| 11. | éther d'alcool octyldodécylique polyéthoxylé (addition de 20 moles) | 8,00 |
| 12. | polysaccharide B-16 (A-2) | 0,02 |
| 13. | méthylparabène | 0,05 |
| 14. | eau pure | 12,63 |

(Exemple comparatif 1 : crème retenant l'humidité 7)

On obtient une crème retenant l'humidité 7 par le même procédé que celui de l'exemple 1, sauf que l'on remplace 0,01 % de polysaccharide A-1 (le composant de mélange N˚ 10) et 7,99 % d'eau pure dans les composants de mélange de l'exemple 1 par 3,00 % de stéarate polyéthoxylé (addition de 40 moles) et 5,00 % de monooléate de sorbitan polyéthoxylé (addition de 15 moles).

(Exemple comparatif 2 : crème retenant l'humidité 8)

On obtient une crème retenant l'humidité 8 par le même procédé que celui de l'exemple 1, sauf que l'on remplace 0,01 % de polysaccharide A-1 (le composant de mélange N˚ 10) et 64,89 % d'eau pure dans les composants de mélange de l'exemple 1 par 0,50 % de gomme xanthane et 64,40 % d'eau pure.

(Exemple comparatif 3 : crème retenant l'humidité 9)

On obtient une crème retenant l'humidité 9 par le même procédé que celui de l'exemple 1, sauf que l'on remplace 0,01 % de polysaccharide A-1 (le composant de mélange N˚ 10) et 4,49 % d'eau pure dans les composants de mélange de l'exemple 1 par 0,50 % de gomme xanthane, 1,00 % de stéarate polyéthoxylé (addition de 40 moles) et 3,00 % de monooléate de sorbitan polyéthoxylé (addition de 15 moles).

(Exemple comparatif 4 : crème de blanchiment 2)

On obtient une crème de blanchiment 2 par le même procédé que celui de l'exemple 7, sauf que l'on remplace 0,10 % de polysaccharide A-1 (le composant de mélange N˚ 12) et 0,40 % d'eau pure dans les composants de mélange de l'exemple 7 par 0,50 % de gélatine.

(Exemple comparatif 5 : crème solaire 2)

On obtient une crème solaire 2 par le même procédé que celui de l'exemple 8, sauf que l'on remplace 0,10 %

de polysaccharide A-2 (le composant de mélange N° 12) et 0,40 % d'eau pure dans les composants de mélange de l'exemple 8 par 0,50 % de méthylcellulose.

(Exemple comparatif 6 : crème de maquillage pour les yeux 2)

On obtient une crème de maquillage pour les yeux 2 par le même procédé que celui de l'exemple 9, sauf que l'on remplace 0,10 % de polysaccharide A-2 (le composant de mélange N° 16) et 0,40 % d'eau pure dans les composants de mélange de l'exemple 9 par 0,50 % de gomme gellane.

(Exemple comparatif 7 : crème de fond de teint 2)

On obtient une crème de fond de teint 2 par le même procédé que celui de l'exemple 10, sauf que l'on remplace 0,10 % de polysaccharide A-2 (le composant de mélange N° 13) et 0,40 % d'eau pure dans les composants de mélange de l'exemple 10 par 0,50 % de gomme agar.

(Exemple comparatif 8 : rouge pour les joues 2)

On obtient du rouge pour les joues 2 par le même procédé que celui de l'exemple 11, sauf que l'on remplace 0,10 % de polysaccharide B-16 A-1 (le composant de mélange N° 16) et 0,40 % d'eau pure dans les composants de mélange de l'exemple 11 par 0,25 % de gélatine et 0,25 % de gomme xanthane.

(Exemple comparatif 9 : rouge à lèvre 2)

On obtient du rouge à lèvre 2 par le même procédé que celui de l'exemple 12, sauf que l'on remplace 0,10 % de polysaccharide A-1 (le composant de mélange N° 17) et 0,40 % d'eau pure dans les composants de mélange de l'exemple 12 par 0,25 % de gomme xanthane et 0,25 % de méthylcellulose.

(Exemple comparatif 10 : crème de nettoyage 2)

On obtient la crème de nettoyage 2 de l'exemple comparatif 10 par le même procédé que celui de l'exemple 13, sauf que l'on remplace 0,10 % de polysaccharide A-1 (le composant de mélange N° 11) et 0,40 % d'eau pure dans les composants de mélange de l'exemple 13 par 0,25 % de gomme xanthane et 0,25 % de gomme gellane.

(Exemple comparatif 11 : crème capillaire 2)

On obtient la crème capillaire 2 par le même procédé que celui de l'exemple 14, sauf que l'on remplace 0,10 % de polysaccharide A-1 (le composant de mélange N° 10) et 0,40 % d'eau pure dans les composants de mélange de l'exemple 14 par 0,25 % de gomme xanthane et 0,25 % de gomme agar.

(Exemple comparatif 12 : gel de nettoyage 2)

On obtient le gel de nettoyage 2 par le même procédé que celui de l'exemple 15, sauf que l'on remplace 0,10 % de polysaccharide A-1 (le composant de mélange N° 6) et 0,40 % d'eau pure dans les composants de mélange de l'exemple 15 par 0,25 % de gomme gellane et 0,25 % de gomme agar.

(Exemple comparatif 13 : gel retenant l'humidité 2)

On obtient le gel retenant l'humidité 2 par le même procédé que celui de l'exemple 16, sauf que l'on remplace 0,10 % de polysaccharide A-1 (le composant de mélange N° 5) et 0,40 % d'eau pure dans les composants de mélange de l'exemple 16 par 0,20 % de gomme xanthane.

(Exemple comparatif 14 : gel de plâtre 2)

On obtient le gel de plâtre 2 par le même procédé que celui de l'exemple 17, sauf que l'on remplace 0,10 % de polysaccharide A-1 (le composant de mélange N° 6) et 0,40 % d'eau pure dans les composants de mélange de l'exemple 17 par 0,20 % de gomme xanthane.

[Teneur en mousse des produits cosmétiques en mousse et essai de stabilité mousse]

On mesure le poids de 100 ml de chaque produit cosmétique en mousse obtenu avec les rapports de mélange décrits ci-dessus dans un cylindre de mesure fermé hermétiquement de 100 ml, et on calcule la teneur en mousse (%) par l'équation suivante:

$$\text{Teneur en mousse (\%)} = [(\beta - \alpha)/\beta] \times 100$$

dans laquelle $\alpha$ est le volume (ml) de 100 g de produits cosmétiques en mousse avant un traitement au fouet, et $\beta$ est le volume (ml) de 100 g de produits cosmétiques en mousse après un traitement au fouet.

Ensuite, on ferme hermétiquement 100 ml de chaque produit cosmétique dans un cylindre de mesure de 100 ml, et on le place dans une chambre thermostatée à 40°C. Au bout de quatre semaines, on mesure le volume de chaque produit cosmétique dans le cylindre de mesure fermé hermétiquement de 100 ml, qui a diminué en raison de l'affaissement et de la coalescence de la mousse. Il est préférable que la réduction de volume soit plus faible. Les résultats de mesure sont présentés dans le tableau 1.

[Evaluation de la sensation des produits cosmétiques à l'emploi]

On applique les produits cosmétiques en mousse, obtenus avec les rapports de mélange décrits ci-dessus, sur le dos de la main de dix femmes pour réaliser un essai sensoriel. Les critères d'évaluation sont les suivants :

* Essai sensoriel 1 :
(+) le produit cosmétique peut être pris à la main avec une sensation douce et légère,
(-) le produit cosmétique ne peut pas être pris à la main avec une sensation douce et légère.
* Essai sensoriel 2:
(+) le produit cosmétique peut être étalé sur la peau avec une sensation légère,
(-) le produit cosmétique ne peut pas être étalé sur la peau avec une sensation légère.
* Essai sensoriel 3 :
(+) le produit cosmétique peut présenter une sensation fraîche après emploi,
(-) le produit cosmétique ne peut pas présenter une sensation fraîche après emploi.

Lorsque six experts ou plus jugent le produit cosmétique (+), il est évalué "O", et lorsque cinq experts ou moins le jugent (+), il est évalué "X". Les résultats d'évaluation sont présentés dans le tableau 1.

Les produits cosmétiques en crème et/ou en gel préparés par le procédé de la présente invention contiennent une mousse homogène de façon à donner un produit cosmétique en crème fouettée et/ou en mousse présentant une sensation douce et légère.

[Tableau 1]

| Ex. | N° | Teneur en mouse immédiatement après la production des produits cosmétiques en mousse (%) | Volume réduit des produits cosmétiques en mousse (ml) | Résultats des essais sensoriels | | |
|---|---|---|---|---|---|---|
| | | | | Essai sensoriel 1 | Essai sensoriel 2 | Essai sensoriel 3 |
| | 1 | 52 | 2 | ○ | ○ | ○ |
| | 2 | 53 | 1 | ○ | ○ | ○ |
| | 3 | 54 | 0 | ○ | ○ | ○ |
| | 4 | 53 | 0 | ○ | ○ | ○ |
| | 5 | 56 | 0 | ○ | ○ | ○ |
| | 6 | 59 | 0 | ○ | ○ | ○ |
| | 7 | 56 | 0 | ○ | ○ | ○ |
| | 8 | 47 | 0 | ○ | ○ | ○ |
| | 9 | 40 | 0 | ○ | ○ | ○ |
| | 10 | 36 | 1 | ○ | ○ | ○ |
| | 11 | 46 | 1 | ○ | ○ | ○ |
| | 12 | 43 | 0 | ○ | ○ | ○ |
| | 13 | 52 | 1 | ○ | ○ | ○ |
| | 14 | 38 | 0 | ○ | ○ | ○ |
| | 15 | 52 | 0 | ○ | ○ | ○ |
| | 16 | 48 | 0 | ○ | ○ | ○ |
| | 17 | 53 | 0 | ○ | ○ | ○ |
| | 18 | 5 | 0 | ○ | ○ | ○ |
| | 19 | 16 | 1 | ○ | ○ | ○ |
| | 20 | 34 | 1 | ○ | ○ | ○ |
| | 21 | 28 | 2 | ○ | ○ | ○ |
| | 22 | 37 | 1 | ○ | ○ | ○ |
| | 23 | 32 | 0 | ○ | ○ | ○ |
| | 24 | 42 | 1 | ○ | ○ | ○ |

(suite)

| Ex. | N° | Teneur en mouse immédiatement après la production des produits cosmétiques en mousse (%) | Volume réduit des produits cosmétiques en mousse (ml) | Résultats des essais sensoriels | | |
|---|---|---|---|---|---|---|
| | | | | Essai sensoriel 1 | Essai sensoriel 2 | Essai sensoriel 3 |
| | 1 | 58 | 32 | X | X | X |
| | 2 | 32 | 28 | X | X | X |
| | 3 | 55 | 21 | X | X | X |
| | 4 | 47 | 29 | X | X | X |
| | 5 | 44 | 25 | X | X | X |
| | 6 | 43 | 21 | X | X | X |
| | 7 | 46 | 18 | X | X | X |
| | 8 | 43 | 18 | X | X | X |
| | 9 | 52 | 19 | X | X | X |
| | 10 | 39 | 21 | X | X | X |
| | 11 | 52 | 17 | X | X | X |
| | 12 | 44 | 21 | X | X | X |
| | 13 | 52 | 21 | X | X | X |
| | 14 | 56 | 28 | X | X | X |

**Revendications**

1. Procédé de stabilisation d'une mousse dans des produits cosmétiques comprenant une étape de mélange d'un polysaccharide constitué au moins de fucose, de glucose, d'acide glucuronique et de rhamnose en tant que monosaccharides constitutifs.

2. Procédé de stabilisation d'une mousse dans des produits cosmétiques selon la revendication 1, dans lequel ledit polysaccharide est un produit d'un micro-organisme de la souche Alcaligenes latus B-16.

3. Procédé de stabilisation d'une mousse dans des produits cosmétiques selon la revendication 1 ou 2, dans lequel ledit polysaccharide est mélangé dans une proportion de 0,01 à 0,5 % en poids, convertie en fraction solide séchée, par rapport à quantité totale du produit cosmétique.

**Claims**

1. Method for stabilizing a foam in cosmetics , comprising a step of mixing a polysaccharide consisting at least of fucose, glucose, glucuronic acid, and rhamnose as constituting monosaccharides.

2. Method for stabilizing a foam in cosmetics according to claim 1, in which said polysaccharide is a product of a microorganism of the strain Alkaligenes latus B-16.

3. Method for stabilizing a foam in cosmetics according to claim 1 or 2, in which said polysaccharide is mixed in a proportion of 0.01 to 0.5 weight %, as a dry solid fraction relative to the total amount of the cosmetic product.

## Patentansprüche

1. Verfahren zur Stabilisierung eines Schaums in kosmetischen Produkten, mit einem Verfahrensschritt des Mischens eines Polysaccharids das zumindest aus Fukose, Glukose, Glucuronsäure und Rhamnose als anteilige Monosaccharide besteht.

2. Verfahren zur Stabilisierung eines Schaums in kosmetischen Produkten nach Anspruch 1, in dem das besagte Polysaccharid ein Produkt eines Mikroorganismus des Stammes Alkaligenes Latus B-16 ist.

3. Verfahren zur Stabilisierung eines Schaums in kosmetischen Produkte nach Anspruch 1 oder 2, in dem das besagte Polysaccharid im Verhältnis 0,01 bis 0,5 Gew. %, auf festen Trockenanteil umgewandelt, der Menge des gesamten kosmetischen Produkts vermischt wird.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- JP 63023962 B
- JP 8503936 A
- JP 2000355517 A


### Littérature non-brevet citée dans la description


- le Japan Agricultural Chemistry Society. *the Year Large Meeting,* 1998, 371
- **Y. NOHATA ; J. AZUMA ; R. KURANE.** *Carbohydrate Research,* 1996, vol. 293, 213-222